# EUROPEAN PATENT SPECIFICATION

(11) **EP 3 415 585 B1**
(45) Date of publication and mention of the grant of the patent: **28.04.2021**
(21) Application number: 17875864.5
(22) Date of filing: 27.11.2017
(51) Int. Cl.: C09K 11/06, C07D 405/14, C07D 409/14, H01L 51/54

(54) **ORGANIC LIGHT-EMITTING ELEMENT**
ORGANISCHES LICHTEMITTIERENDES ELEMENT
ÉLÉMENT ÉLECTROLUMINESCENT ORGANIQUE

(30) Priority: 29.11.2016 KR 20160160371; 17.11.2017 KR 20170154011
(43) Date of publication of application: 19.12.2018
(73) Proprietor: LG Chem, Ltd., Seoul 07336 (KR)
(72) Inventor: CHO, Seong Mi, Daejeon 34122 (KR); LEE, Jung Ha, Daejeon 34122 (KR); LEE, Dong Hoon, Daejeon 34122 (KR); PARK, Tae Yoon, Daejeon 34122 (KR); MUN, Jeong Wook, Daejeon 34122 (KR); JUNG, Min Woo, Daejeon 34122 (KR); LEE, Ju Young, Daejeon 34122 (KR)
(74) Representative: Hoffmann Eitle
(86) International application number: PCT/KR2017/013613
(87) International publication number: WO 2018/101691

(56) References cited:
- WO-A1-2015/014434
- WO-A1-2015/051869
- WO-A1-2016/129672
- WO-A1-2016/129672
- KR-A- 20150 003 051
- KR-A- 20150 071 624
- KR-A- 20160 026 744
- KR-A- 20160 054 582
- TW-A- 201 533 038

## Description

### [TECHNICAL FIELD]

### CROSS-REFERENCE TO RELATED APPLICATION(S)

This application claims priority to and the benefit of Korean Patent Application No. 10-2016-0160371 filed on November 29, 2016 and Korean Patent Application No. 10-2017-0154011 filed on November 17, 2017 with the Korean Intellectual Property Office.

The present invention relates to an organic light emitting device having improved driving voltage, efficiency and lifetime.

### [BACKGROUND ART]

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently have a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer and the like. In the structure of the organic light emitting device, if a voltage is applied between two electrodes, the holes are injected from an anode into the organic material layer and the electrons are injected from the cathode into the organic material layer, and when the injected holes and the electrons meet each other, an exciton is formed, and light is emitted when the exciton falls to a ground state again.

There is a continuing demand for developing an organic light emitting device having improved driving voltage, efficiency and lifetime.

### [Prior Art Literature]

### [Patent Literature]

(Patent Literature 1) Korean Patent Laid-open Publication No. 10-2000-0051826

TW201533038 A, WO2015/014434 A1, and WO2015/051869 A1 disclose organic light emitting devices comprising multiple hosts in the light emitting layer. WO2016/129672 A1 discloses an organic light emitting device comprising a single host in its light emitting layer.

### [DETAILED DESCRIPTION OF THE INVENTION]

### [Technical Problem]

It is one object of the present invention to provide an organic light emitting device having improved driving voltage, efficiency and lifetime.

### [Technical Solution]

The present invention provides an organic light emitting device comprising:
an anode; a cathode; and at least one light emitting layer interposed between the anode and the cathode,
wherein the light emitting layer comprises a first host compound represented by Chemical Formulas 1-1, 1-2, or 1-3 of claim 1, and a second host compound represented by Chemical Formula 2 of claim 1
R₁ is a substituted or unsubstituted C₁₋₆₀ alkyl; a substituted or unsubstituted C₃₋₆₀ cycloalkyl; a substituted or unsubstituted C₆₋₆₀ aryl; or a substituted or unsubstituted C₆₋₆₀ heteroaryl containing at least one of O, N, Si and S,
R₂ and R₃ are each independently hydrogen; deuterium; halogen; cyano; a substituted or unsubstituted C₁₋₆₀ alkyl; or a substituted or unsubstituted C₃₋₆₀ cycloalkyl,
n is an integer of 0 to 4,
m is an integer of 0 to 3,
Y is O, S, or CR₄R₅,
R₄ and R₅ are each independently hydrogen; deuterium; halogen; cyano; a substituted or unsubstituted C₁₋₆₀ alkyl; a substituted or unsubstituted C₃₋₆₀ cycloalkyl; or a substituted or unsubstituted C₆₋₆₀ aryl,
Ar is represented by the following Chemical Formula 1' 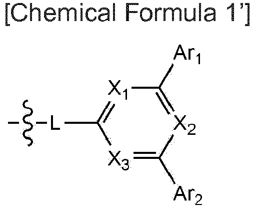
wherein,
L is a single bond; a substituted or unsubstituted C₆₋₆₀ arylene; or a substituted or unsubstituted C₂₋₆₀ heteroarylene containing at least one of O, N, Si and S,
X₁ to X₃ are each independently N, or CR₆, provided that at least one of X₁ to X₃ is N,
each R₆ is independently hydrogen; deuterium; halogen; cyano; nitro; amino; a substituted or unsubstituted C₁₋₆₀ alkyl; a substituted or unsubstituted C₁₋₆₀ haloalkyl; a substituted or unsubstituted C₁₋₆₀ haloalkoxy; a substituted or unsubstituted C₃₋₆₀ cycloalkyl; a substituted or unsubstituted C₂₋₆₀ alkenyl; a substituted or unsubstituted C₆₋₆₀ aryl; or a substituted or unsubstituted C₂₋₆₀ heteroaryl containing at least one of O, N, Si and S,
Ar₁ and Ar₂ are each independently a substituted or unsubstituted C₆₋₆₀ aryl; or a substituted or unsubstituted C₂₋₆₀ heteroaryl containing at least one of O, N, Si and S, 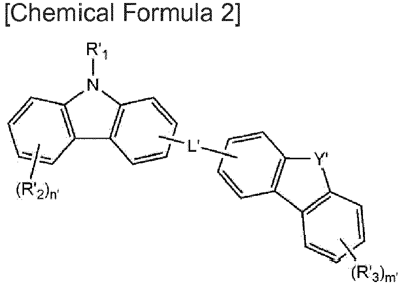
in Chemical Formula 2,
R'₁ is cyclohexyl, phenyl, cyano-substituted phenyl, biphenylyl, terphenylyl, naphthyl, phenanthryl, triphenylenyl, dimethylfluorenyl, pyridinyl, dibenzofuranyl, dibenzothiophenyl, or 9-phenyl-carbazolyl,
R'₂ and R'₃ are each independently hydrogen; deuterium; halogen; cyano; a substituted or unsubstituted C₁₋₆₀ alkyl; a substituted or unsubstituted C₃₋₆₀ cycloalkyl; a substituted or unsubstituted C₆₋₆₀ aryl; or a substituted or unsubstituted C₂₋₆₀ heteroaryl containing at least one of O, N, Si and S,
n' and m' are each independently an integer of 0 to 4,
L' is a single bond; or a substituted or unsubstituted C₆₋₆₀ arylene,
Y' is O, S, NR', or CR'R",
R' and R" are each independently a substituted or unsubstituted C₁₋₆₀ alkyl; a substituted or unsubstituted C₃₋₆₀ cycloalkyl; or a substituted or unsubstituted C₆₋₆₀ aryl; or R' and R" together form a substituted or unsubstituted C₆₋₆₀ aromatic ring,
and wherein the first host compound and the second host compound are different from each other.

### [ADVANTAGEOUS EFFECTS]

The organic light emitting device described above is excellent in driving voltage, efficiency, and lifetime.

### [BRIEF DESCRIPTION OF DRAWINGS]

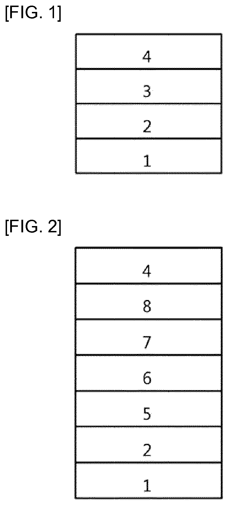

FIG. 1 shows an example of an organic light emitting device comprising a substrate 1, an anode 2, a light emitting layer 3, and a cathode 4.
FIG. 2 shows an example of an organic light emitting device comprising a substrate 1, an anode 2, a hole injection layer 5, a hole transport layer 6, a light emitting layer 7, an electron transport layer 8, and a cathode 4.

### [DETAILED DESCRIPTION OF THE EMBODIMENTS]

Hereinafter, the present invention will be described in more detail to help understanding of the present invention.

In the present specification, 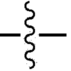 means a bond connected to another substituent group.

As used herein, the term "substituted or unsubstituted" means that substitution is performed by one or more substituent groups selected from the group consisting of deuterium; a halogen group; a nitrile group; a nitro group; a hydroxyl group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; or a heterocyclic group containing at least one of N, O, and S atoms, or there is no substituent group, or substitution is performed by a substituent group where two or more substituent groups of the exemplified substituent groups are linked or there is no substituent group. For example, the term "substituent group where two or more substituent groups are linked" may be a biphenyl group. That is, the biphenyl group may be an aryl group, or may be interpreted as a substituent group where two phenyl groups are connected.

In the present specification, the number of carbon atoms in a carbonyl group is not particularly limited, but is preferably 1 to 40 carbon atoms. Specifically, the carbonyl group may be compounds having the following structures, but is not limited thereto.

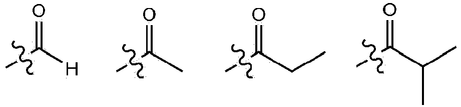

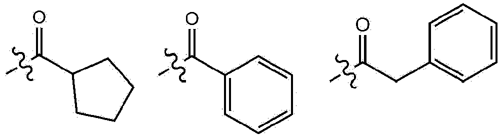

In the present specification, the ester group may have a structure in which oxygen of the ester group may be substituted by a straight-chain, branched-chain, or cyclic alkyl group having 1 to 25 carbon atoms, or an aryl group having 6 to 25 carbon atoms. Specifically, the ester group may be compounds having the following structures, but is not limited thereto.

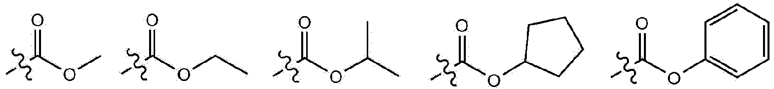

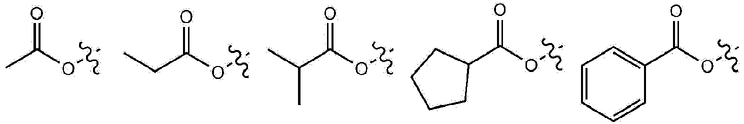

In the present specification, the number of carbon atoms in an imide group is not particularly limited, but is preferably 1 to 25. Specifically, the imide group may be compounds having the following structures, but is not limited thereto.

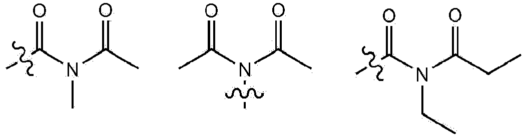

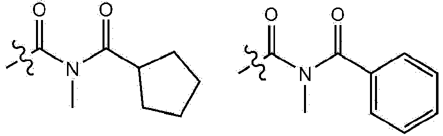

In the present specification, the silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but is not limited thereto.

In the present specification, the boron group specifically includes a trimethylboron group, a triethylboron group, a t-butyldimethylboron group, a triphenylboron group, a phenylboron group, and the like, but is not limited thereto.

In the present specification, examples of a halogen group include fluorine, chlorine, bromine, or iodine.

In the present specification, an alkyl group may be a straight chain or a branched chain, and the number of carbon atoms thereof is not particularly limited, but is preferably 1 to 40. According to one embodiment, the alkyl group has 1 to 20 carbon atoms. According to another embodiment, the alkyl group has 1 to 10 carbon atoms. According to still another embodiment, the alkyl group has 1 to 6 carbon atoms. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, and the like, but are not limited thereto.

In the present specification, the alkenyl group may be a straight chain or a branched chain, and the number of carbon atoms thereof is not particularly limited, but is preferably 2 to 40. According to one embodiment, the alkenyl group has 2 to 20 carbon atoms. According to another embodiment, the alkenyl group has 2 to 10 carbon atoms. According to still another embodiment, the alkenyl group has 2 to 6 carbon atoms. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present specification, a cycloalkyl group is not particularly limited, but the number of carbon atoms thereof is preferably 3 to 60. According to one embodiment, the cycloalkyl group has 3 to 30 carbon atoms. According to another embodiment, the cycloalkyl group has 3 to 20 carbon atoms. According to another embodiment, the cycloalkyl group has 3 to 6 carbon atoms. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present specification, the aryl group is not particularly limited, but preferably has 6 to 60 carbon atoms, and may be a monocyclic aryl group or a polycyclic aryl group. According to one embodiment, the aryl group has 6 to 30 carbon atoms. According to one embodiment, the aryl group has 6 to 20 carbon atoms. The aryl group may be a phenyl group, a biphenyl group, a terphenyl group or the like as the monocyclic aryl group, but is not limited thereto. Examples of the polycyclic aryl group include a naphthyl group, an anthracenyl group, a phenanthryl group, a pyrenyl group, a perylenyl group, a chrycenyl group, a fluorenyl group or the like, but is not limited thereto.

In the present specification, a fluorenyl group may be substituted, and two substituent groups may be linked with each other to form a spiro structure. In the case where the fluorenyl group is substituted, 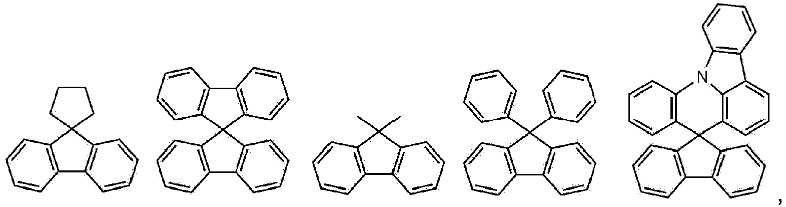 and the like can be formed. However, the structure is not limited thereto.

In the present specification, the heterocyclic group is a heterocyclic group containing at least one of O, N, Si and S as a heteroatom, and the number of carbon atoms thereof is not particularly limited, but is preferably 2 to 60. Examples of the heterocyclic group include a thiophene group, a furan group, a pyrrole group, an imidazole group, a triazole group, an oxazole group, an oxadiazole group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzimidazole group, a benzothiazole group, a benzocarbazole group, a benzothiophene group, a dibenzothiophene group, a benzofuranyl group, a phenanthroline group, an isoxazolyl group, a thiadiazolyl group, a phenothiazinyl group, a dibenzofuranyl group, and the like, but are not limited thereto.

In the present specification, the aryl group in the aralkyl group, the aralkenyl group, the alkylaryl group, and the arylamine group is the same as the aforementioned examples of the aryl group. In the present specification, the alkyl group in the aralkyl group, the alkylaryl group and the alkylamine group is the same as the aforementioned examples of the alkyl group. In the present specification, the heteroaryl in the heteroarylamines can be applied to the aforementioned description of the heterocyclic group. In the present specification, the alkenyl group in the aralkenyl group is the same as the aforementioned examples of the alkenyl group. In the present specification, the aforementioned description of the aryl group may be applied except that the arylene is a divalent group. In the present specification, the aforementioned description of the heterocyclic group can be applied except that the heteroarylene is a divalent group. In the present specification, the aforementioned description of the aryl group or cycloalkyl group can be applied except that the hydrocarbon ring is not a monovalent group but formed by combining two substituent groups. In the present specification, the aforementioned description of the heterocyclic group can be applied, except that the heterocycle is not a monovalent group but formed by combining two substituent groups.

The present invention provides the following organic light emitting device:
An organic light emitting device comprising: an anode; a cathode; and at least one light emitting layer interposed between the anode and the cathode, wherein the light emitting layer comprises a first host compound represented by Chemical Formulas 1-1, 1-2 or 1-3 of claim 1 and a second host compound represented by Chemical Formula 2 of claim 1, wherein the first host compound and the second host compound are different from each other.

Hereinafter, the present invention will be described in detail for each configuration.

### Anode and Cathode

The anode and cathode used in the present invention are electrodes used in an organic light emitting device.

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:AI or SNO₂:Sb; conductive polymers such as poly(3-methylthiophene), poly[3,4-(ethylene-1,2-dioxy)thiophene](PEDOT), polypyrrole, and polyaniline, and the like, but are not limited thereto.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/AI or LiO₂/Al, and the like, but are not limited thereto.

In addition, a hole injection layer may be further included on the anode. The hole injection layer is composed of a hole injection material, and the hole injection material is preferably a compound which has an ability of transporting the holes, a hole injection effect in the anode and an excellent hole injection effect to the light emitting layer or the light emitting material, prevents movement of an exciton generated in the light emitting layer to the electron injection layer or the electron injection material, and has an excellent thin film forming ability.

It is preferable that a HOMO (highest occupied molecular orbital) of the hole injection material is between the work function of the anode material and a HOMO of a peripheral organic material layer. Specific examples of the hole injection material include metal porphyrine, oligothiophene, an arylamine-based organic material, a hexanitrilehexaazatriphenylene-based organic material, a quinacridone-based organic material, a perylene-based organic material, anthraquinone, polyaniline and polythiophene-based conductive polymer, and the like, but are not limited thereto.

### Light Emitting Layer

The light emitting layer according to the present invention includes a first host compound represented by Chemical Formulas 1-1, 1-2 or 1-3 of claim 1 and a second host compound represented by Chemical Formula 2 of claim 1, wherein the first host compound and the second host compound are different from each other.

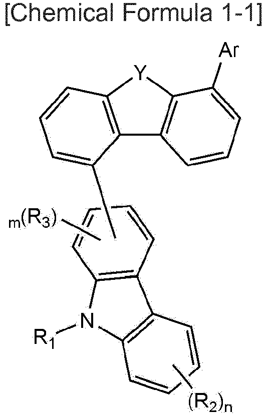

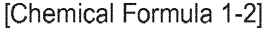

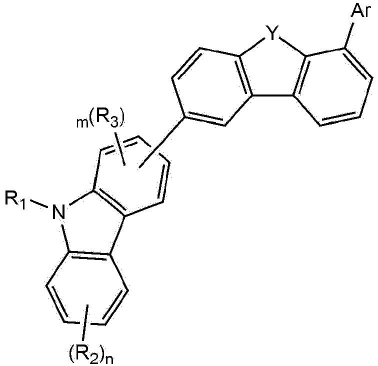

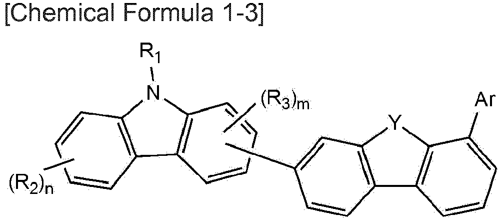

Preferably, R₁ is phenyl; or biphenylyl.

Preferably, R₂ and R₃ are hydrogen.

Preferably, Y is S, or O.

Preferably, L is a single bond, or phenylene.

Preferably, Ar₁ and Ar₂ are each independently phenyl, cyano-substituted phenyl, biphenylyl, dimethylfluorenyl, naphthyl, phenanthryl, pyridinyl, dibenzofuranyl, or dibenzothiophenyl.

Representative examples of the compound represented by Chemical Formulas 1-1, 1-2 or 1-3 are as follows.

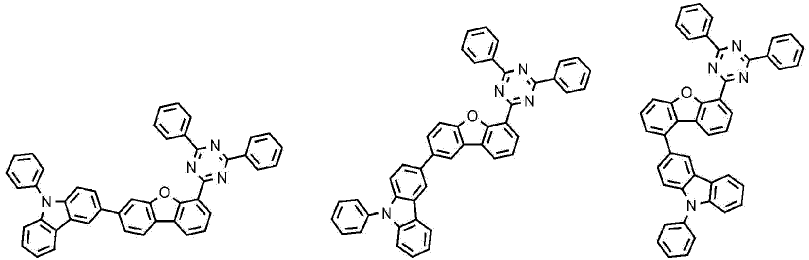

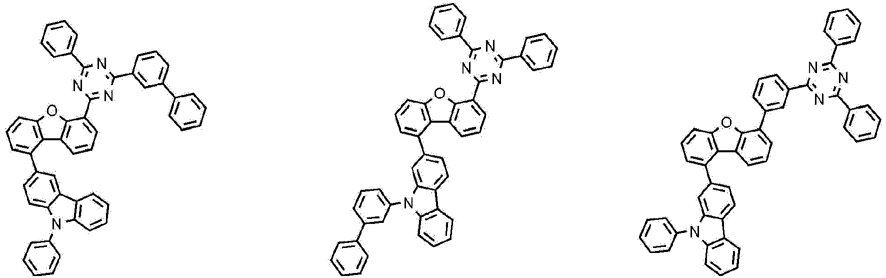

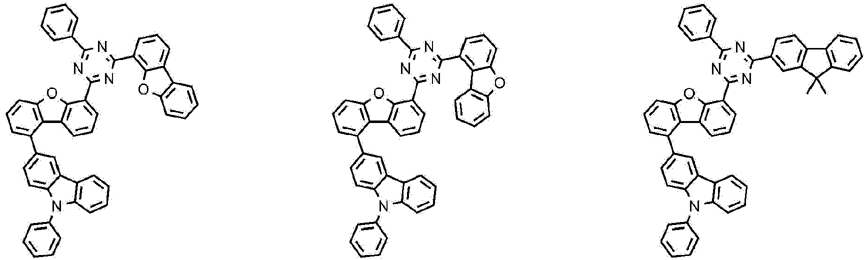

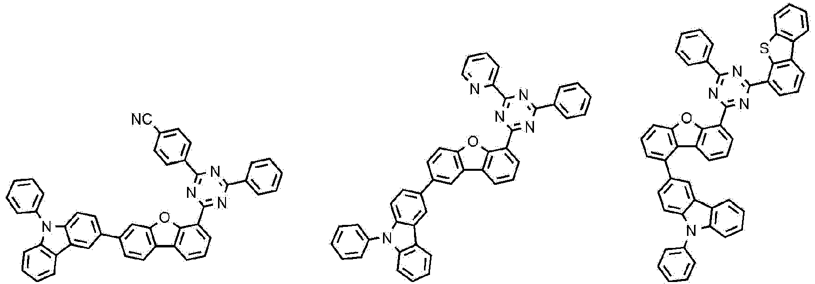

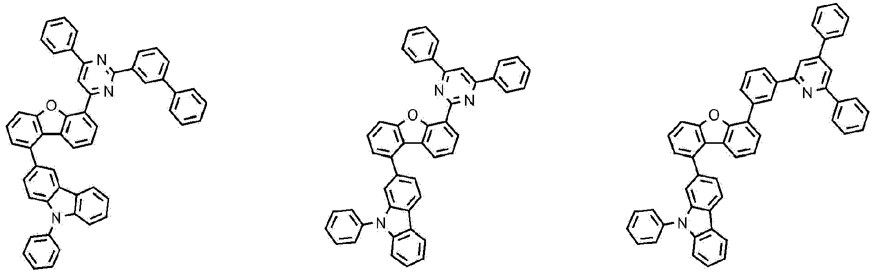

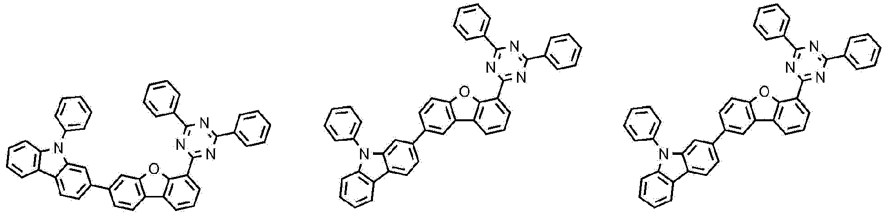

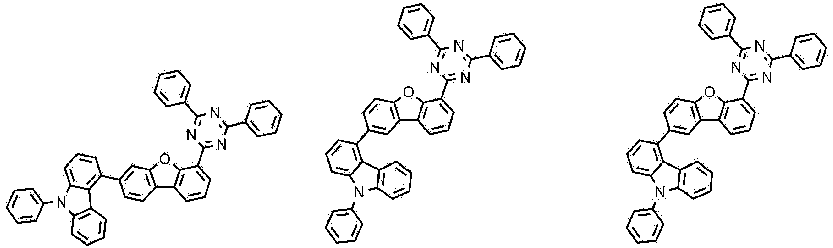

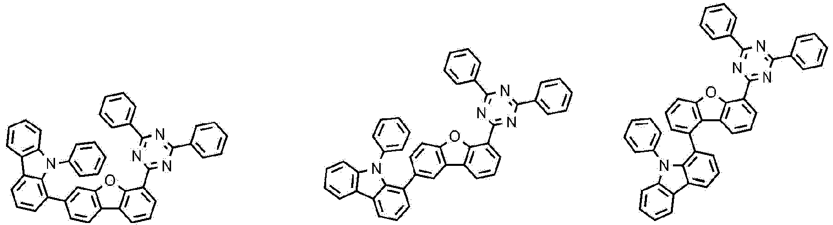

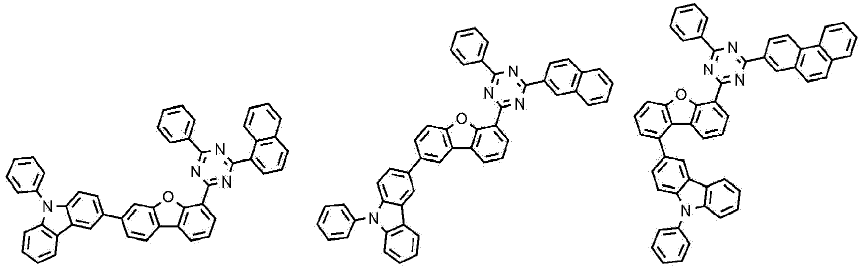

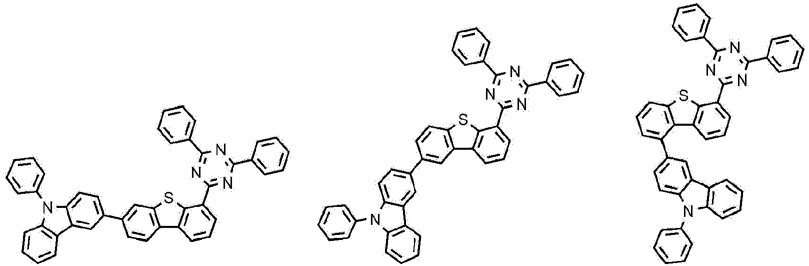

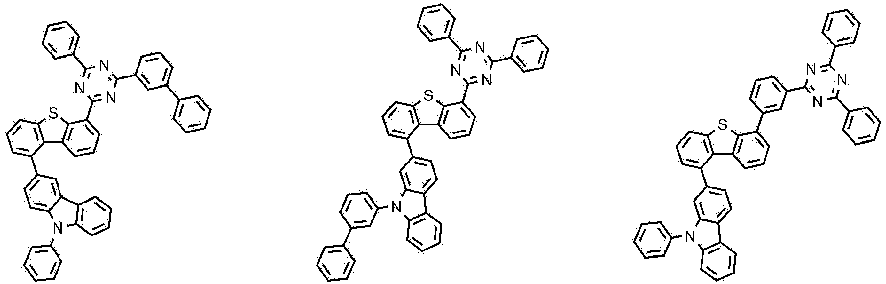

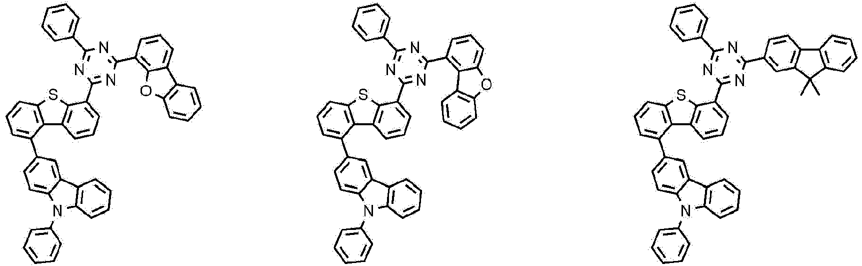

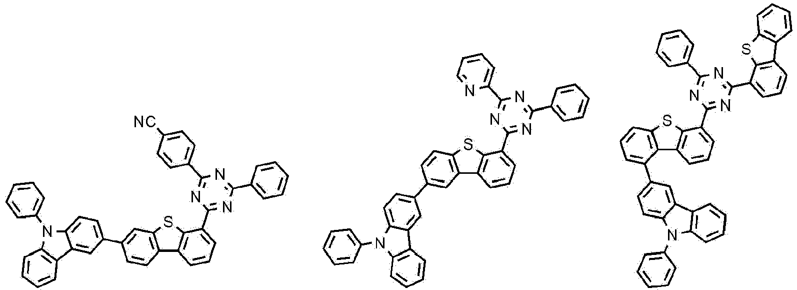

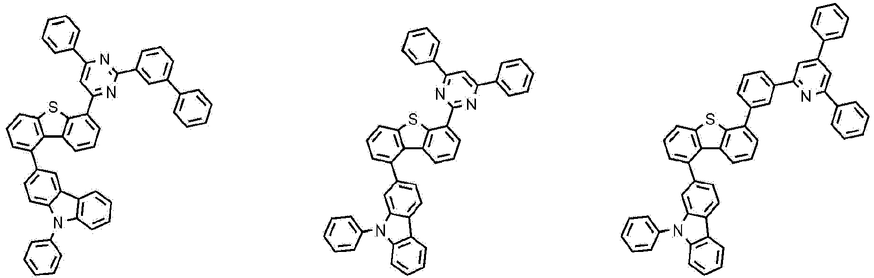

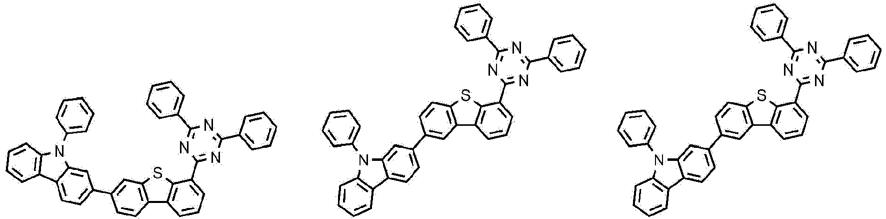

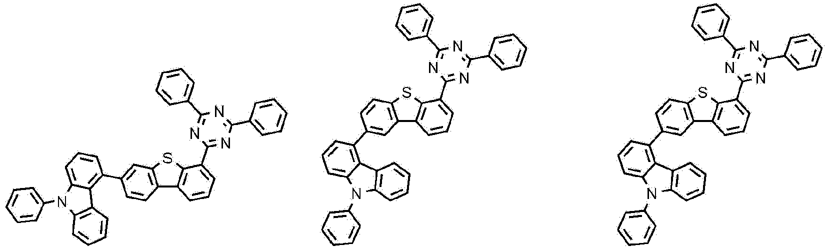

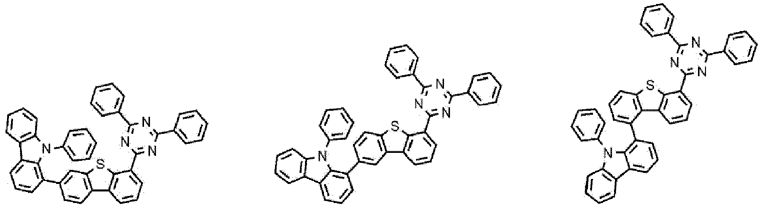

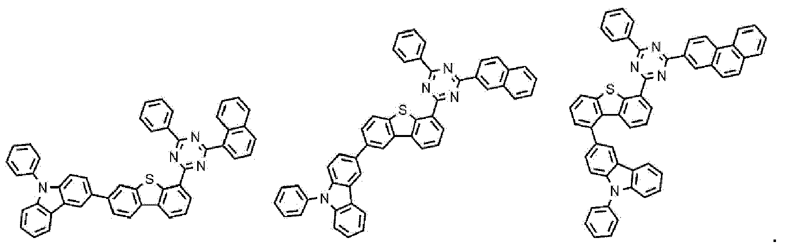

The compound represented by Chemical Formulas 1-1, 1-2 or 1-3 can be prepared, for example, according to the following Reaction Scheme 1.

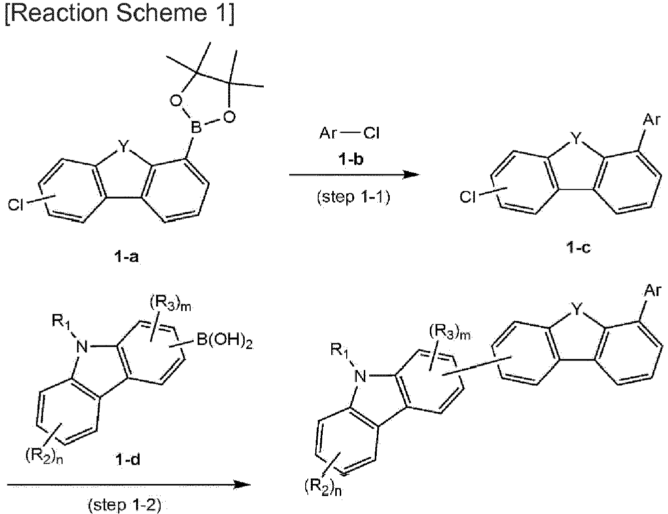

Reaction Scheme 1 is a Suzuki coupling reaction in which each reaction is preferably carried out in the presence of a palladium catalyst and a base. The reactive group for the Suzuki coupling reaction can be modified as known in the art. The above preparation method can be further specified in Preparation Examples to be described later.

In Chemical Formula 2, R'₁ is cyclohexyl, phenyl, cyano-substituted phenyl, biphenylyl, terphenylyl, naphthyl, phenanthryl, triphenylenyl, dimethylfluorenyl, pyridinyl, dibenzofuranyl, dibenzothiophenyl, or 9-phenyl-carbazolyl.

Preferably, n' is 1, and R'₂ is hydrogen, or phenyl.

Preferably, m' is 1, and R'₃ is hydrogen, tert-butyl, cyano, phenyl, cyano-substituted phenyl, or pyridinyl.

Preferably, Y' is O, S, or NR', and R' is phenyl, or biphenylyl.

Preferably, Y' is CR'R", and R' and R" are methyl, or R' and R" together form a fluorene ring.

Preferably, L' is a single bond, or phenylene.

Representative examples of the compound represented by Chemical Formula 2 are as follows:

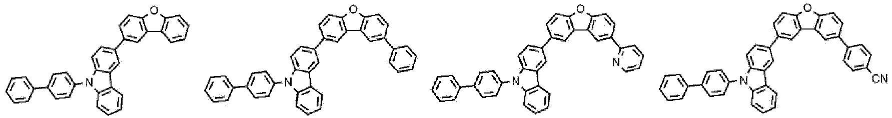

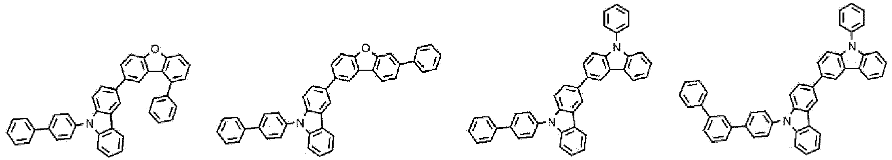

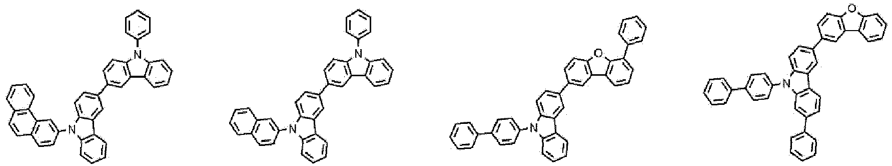

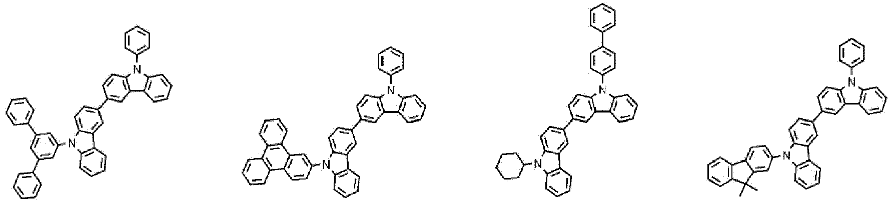

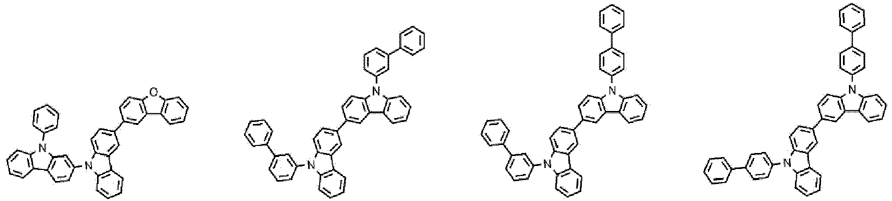

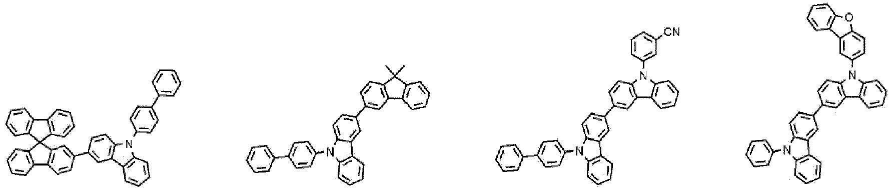

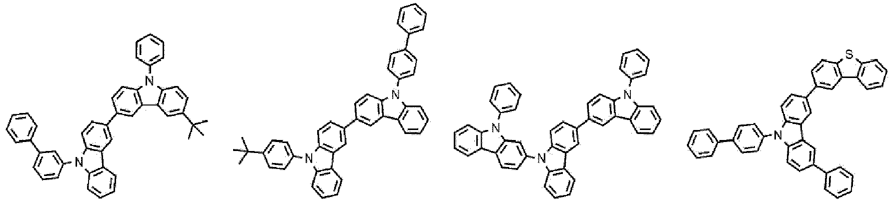

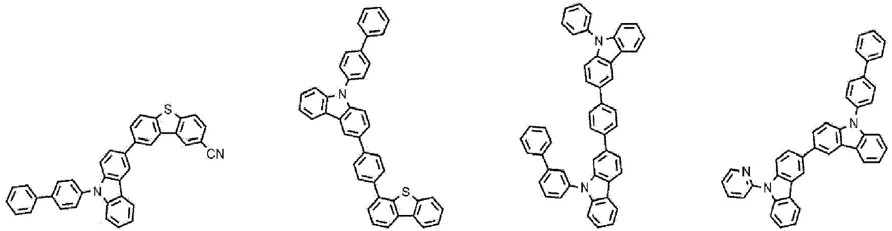

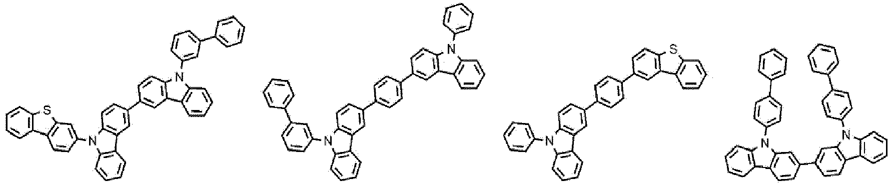

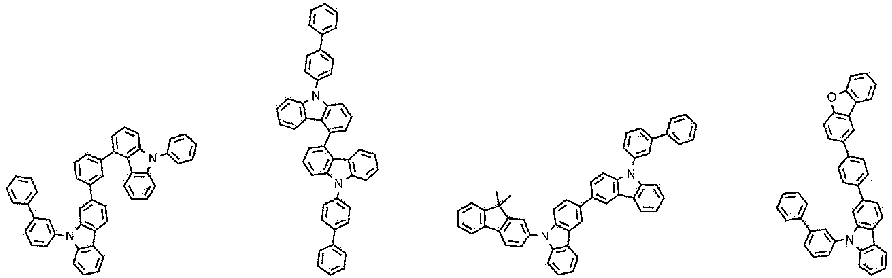

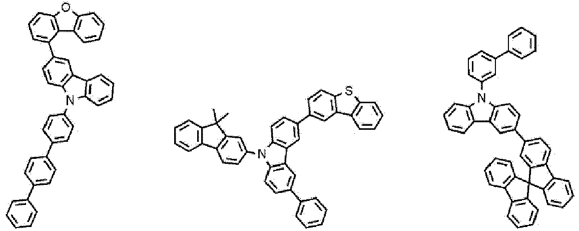

The compound represented by Chemical Formula 2 can be prepared, for example, according to the following Reaction Scheme 2.

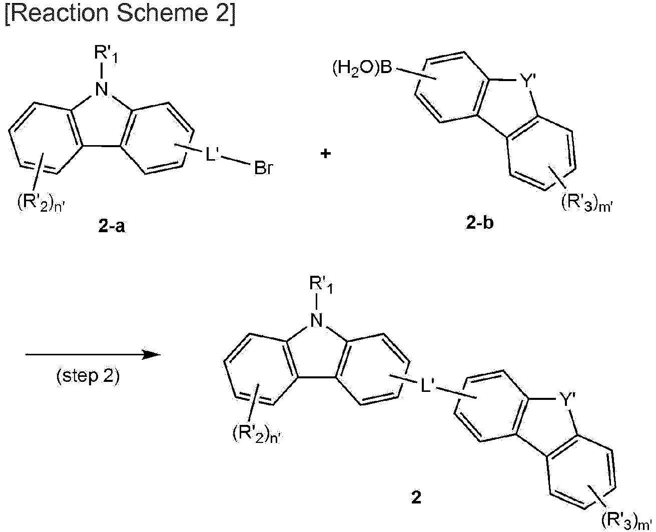

Reaction Scheme 2 is a Suzuki coupling reaction in which each reaction is preferably carried out in the presence of a palladium catalyst and a base. The reactive group for the Suzuki coupling reaction can be modified as known in the art. The above preparation method can be further specified in Preparation Examples to be described later.

Preferably, the weight ratio between the first host compound and the second host compound is 1:99 to 99:1.

In addition, the light emitting layer may include a dopant material in addition to the host compound. The dopant material is not particularly limited as long as it is used for an organic light emitting device, and examples thereof include an aromatic amine derivative, a styrylamine compound, a boron complex, a fluoranthene compound, a metal complex, and the like.

Specifically, the aromatic amine derivative is a condensation aromatic cycle derivative having a substituted or unsubstituted arylamino group, examples thereof include pyrene, anthracene, chrysene, and periflanthene having the arylamino group, and the like, the styrylamine compound is a compound where at least one arylvinyl group is substituted in substituted or unsubstituted arylamine, in which one or two or more substituent groups selected from the group consisting of an aryl group, a silyl group, an alkyl group, a cycloalkyl group, and an arylamino group are substituted or unsubstituted. Specific examples thereof include styrylamine, styryldiamine, styryltriamine, styryltetramine, and the like, but are not limited thereto. Further, examples of the metal complex include an iridium complex, a platinum complex, and the like, but are not limited thereto.

### Other layers

In addition, the organic light emitting device according to the present invention may include a hole injection layer, a hole transport layer, an electron transfer layer, and/or an electron transmission layer, if necessary.

The hole injection material layer is a layer injecting the holes from the electrode, and the hole injection material is preferably a compound which has an ability of transporting the holes, a hole injection effect in the anode and an excellent hole injection effect to the light emitting layer or the light emitting material, prevents movement of an exciton generated in the light emitting layer to the electron injection layer or the electron injection material, and has an excellent thin film forming ability. It is preferable that a HOMO (highest occupied molecular orbital) of the hole injection material is between the work function of the anode material and a HOMO of a peripheral organic material layer. Specific examples of the hole injection material include metal porphyrine, oligothiophene, an arylamine-based organic material, a hexanitrilehexaazatriphenylene-based organic material, a quinacridone-based organic material, a perylene-based organic material, anthraquinone, polyaniline, polythiophene-based conductive polymer, and the like, but are not limited thereto.

The hole transport layer is a layer that can receive the holes from the hole injection layer and transport the holes to the light emitting layer. The hole transport material is suitably a material having large mobility to the holes, which may receive holes from the anode or the hole injection layer and transfer the holes to the light emitting layer. Specific examples thereof include an arylamine-based organic material, a conductive polymer, a block copolymer in which a conjugate portion and a non-conjugate portion are present together, and the like, but are not limited thereto.

The electron transport layer is a layer receiving the electrons from the electron injection layer or the cathode and transporting the electrons to the light emitting layer, the electron transport material is a material that can receive the electrons well from the cathode and transport the electrons to the light emitting layer, and a material having large mobility to the electrons is suitable. Specific examples thereof include an 8-hydroxyquinoline Al complex; a complex including Alq₃; an organic radical compound; a hydroxyflavone-metal complex, and the like, but are not limited thereto. The electron transport layer may be used together with a predetermined desired cathode material as used according to the prior art. Particularly, an example of an appropriate cathode material is a general material having the low work function and followed by an aluminum layer or a silver layer. Specific examples thereof include cesium, barium, calcium, ytterbium, and samarium, and each case is followed by the aluminum layer or the silver layer.

The electron injection layer is a layer injecting the electrons from the electrode, and a compound which has an ability of transporting the electrons, an electron injection effect from the cathode, and an excellent electron injection effect to the light emitting layer or the light emitting material, prevents movement of an exciton generated in the light emitting layer to the hole injection layer, and has an excellent thin film forming ability is preferable. Specific examples thereof include fluorenone, anthraquinodimethane, diphenoquinone, thiopyran dioxide, oxazole, oxadiazole, triazole, imidazole, perylene tetracarboxylic acid, fluorenylidene methane, anthrone, and the like, and its derivative, a metal complex compound, a nitrogen-containing 5-membered cycle derivative, and the like, but are not limited thereto.

Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but are not limited thereto.

### Organic light emitting device

The organic light emitting device according to the present invention can be manufactured by materials and methods known in the art, except that the light emitting layer includes a first host and a second host according to claim 1.

For example, the organic light emitting device according to the present invention may be manufactured by sequentially laminating the anode, the organic material layer and the cathode on the substrate. In this case, the organic light emitting device may be manufactured by depositing a metal, metal oxides having conductivity, or an alloy thereof on the substrate by using a PVD (physical vapor deposition) method such as a sputtering method or an e-beam evaporation method to form the anode, forming the organic material layer including the hole injection layer, the hole transport layer, the light emitting layer, and the electron transport layer thereon, and then depositing a material that can be used as the cathode thereon. In addition to such a method, the organic light emitting device may be manufactured by sequentially depositing a cathode material, the organic material layer, and an anode material on the substrate.

Further, the first host compound and the second compound may be formed as the light emitting layer by a vacuum deposition method as well as a solution coating method during the production of the organic light emitting device. Herein, the solution coating method means spin coating, dip coating, doctor blading, inkjet printing, screen printing, spray method, roll coating, or the like, but is not limited thereto.

In addition to such a method, the organic light emitting device may be manufactured by sequentially depositing an anode material, an organic material layer, and a cathode material on a substrate (International Publication WO 2003/012890). However, the manufacturing method is not limited thereto.

The organic light emitting device according to the present invention may be a front side emission type, a back side emission type, or a double side emission type according to the used material.

Hereinafter, preferred examples of the present invention will be described in order to facilitate understanding of the present invention. However, the following examples are presented for illustrative purposes only, and the scope of the present invention is not limited thereto.

### [Preparation Example]

### Preparation Example 1: Preparation of Intermediate Compound A

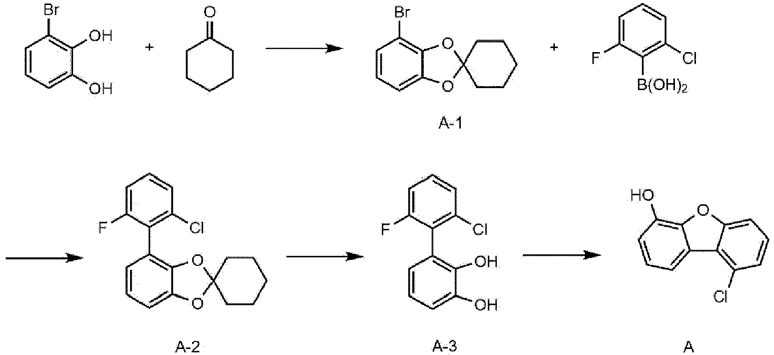

### Step 1: Preparation of Compound A-1

3-Bromobenzene-1,2-diol (100 g, 529 mmol) and cyclohexanone (54.5 g, 555 mmol) were added to toluene and then cooled. Phosphorus trichloride (PCl₃) (29 g, 211.7 mmol) was slowly added thereto at 0°C, and the mixture was stirred at room temperature. When the reaction was completed, the reaction mixture was poured into an excess amount of water, extracted with chloroform, and washed with a saturated aqueous solution of sodium hydrogen carbonate. Then, the organic layer was separated, slurried with anhydrous sodium sulfate, filtered and concentrated under reduced pressure. To the concentrated compound was added hexane and then stirred. The resulting solid was filtered to obtain Compound A-1 (92.6 g, yield: 65%).

### Step 2: Preparation of Compound A-2

Compound A-1 (100 g, 371 mmol) and (2-chloro-6-fluorophenyl)boronic acid (68 g, 390 mmol) were added to 1000 mL of tetrahydrofuran and then cooled. After adding 2M potassium carbonate aqueous solution (aq. K₂CO₃) (370 mL, 743 mmol), tetrakis triphenylphosphine palladium [Pd(PPh₃)₄] (4.3 g, 1 mol%) was added thereto and the mixture was stirred under reflux for 6 hours. After completion of the reaction, the temperature was lowered to room temperature, the water layer was removed, and the organic layer was concentrated under reduced pressure. The concentrated compound was dissolved in 500 mL of chloroform, washed with water and separated. The organic layer was treated with anhydrous magnesium sulfate and filtered. The filtrate was concentrated under reduced pressure, and slurried with an excess amount of hexane and a small amount of ethyl acetate to obtain Compound A-2 (86.5 g, yield: 73%).

### Step 3: Preparation of Compound A-3

Compound A-2 (60 g, 188 mmol) was dispersed in 200 mL of N-methyl-2-pyrrolidone, and 60 mL of hydrochloric acid was added thereto and heated. After reacting for about 2 hours, the reaction mixture was cooled to room temperature, poured into 1 L of water and extracted with ethyl acetate. The organic layer was slurried with saturated sodium hydrogen carbonate and further washed once more with water. The organic layer was slurried with anhydrous magnesium sulfate, filtered and concentrated under reduced pressure. To the concentrated compound was added an excess amount of hexane to produce a solid, which was filtered to prepare Compound A-3 (36.4 g, yield: 81%).

### Step 4: Preparation of Compound A

Compound A-3 (50 g, 210 mmol) was added to 100 mL of N-methyl-2-pyrrolidone under a nitrogen atmosphere and stirred. Potassium carbonate (58 g, 420 mmol) was then added thereto, and the mixture was heated to 80°C and stirred. After completion of the reaction, the temperature was lowered to room temperature, and the reaction mixture was poured into 1 L of water and extracted with ethyl acetate. To the extracted mixture was added anhydrous magnesium sulfate, slurried and filtered, and the filtrate was concentrated under reduced pressure. The concentrated compound was purified by silica column chromatography with hexane and ethyl acetate to obtain Compound A (34.4 g, yield 75%).
MS : [M+ H]⁺=219

### Preparation Example 2: Preparation of Intermediate Compound B

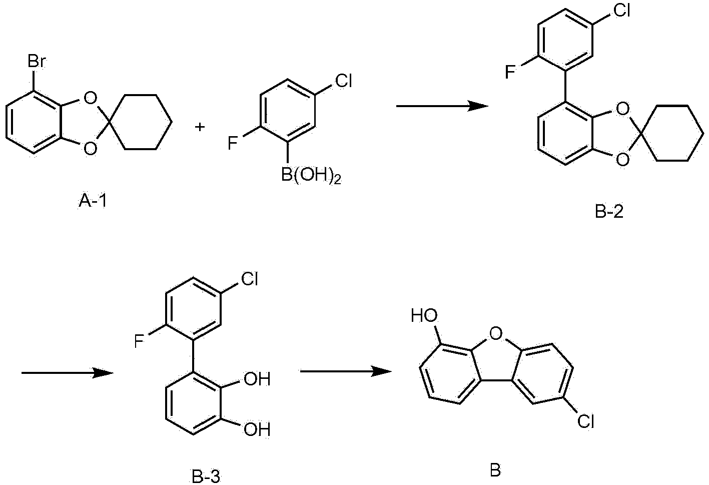

Intermediate Compound B was prepared in the same manner as in Preparation Example of Intermediate Compound A by using Compound A-1 and (5-chloro-2-fluorophenyl)boronic acid.

### Preparation Example 3: Preparation of Intermediate Compound C

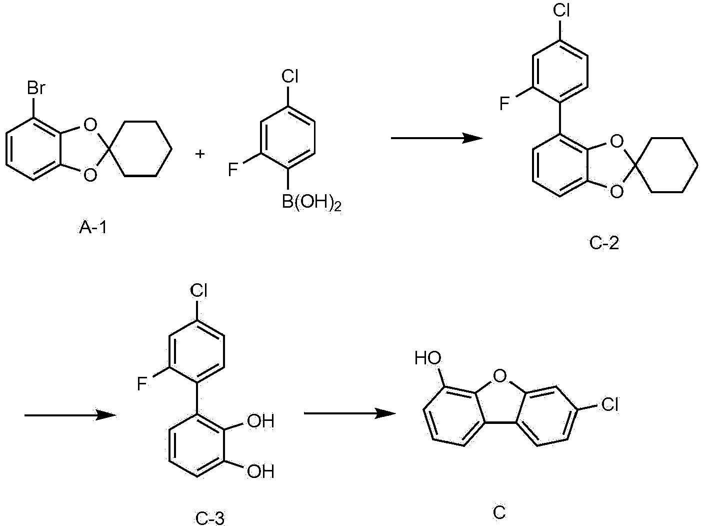

Intermediate Compound C was prepared in the same manner as in Preparation Example of Intermediate Compound A by using Compound A-1 and (4-chloro-2-fluorophenyl)boronic acid.

### Preparation Example 4: Preparation of Intermediate Compound D

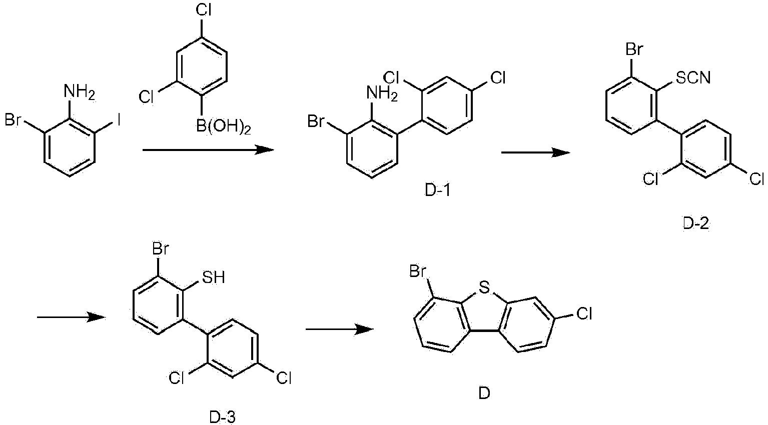

### Step 1) Preparation of Compound D-1

(2,4-Dichlorophenyl)boronic acid (100 g, 524 mmol) and 2-bromo-6-iodoaniline (234 g, 785.5 mmol) were added to 1500 mL of toluene. Then, potassium carbonate (217 g, 1517 mmol) was dissolved in 500 mL of water and added. Tetrakis triphenylphosphine palladium [Pd(PPh₃)₄] (30.3 g, 5 mol%) was added thereto and the mixture was heated and stirred under reflux. After completion of the reaction, the temperature was lowered to room temperature, the water layer was removed, and the organic layer was concentrated under reduced pressure. The concentrated compound was dissolved in 500 mL of chloroform, treated with anhydrous magnesium sulfate and filtered. The filtrate was concentrated under reduced pressure, and then purified by silica column chromatography with hexane and ethyl acetate to obtain Compound D-1 (103.3 g, yield: 61%).

### Step 2: Preparation of Compound D-2

Compound D-1 (101 g, 319.5 mmol) was added to methanol, the temperature was lowered to 0°C, and conc. HCI was slowly added dropwise. Sodium nitrite (NaNO₂) (22 g, 319.5 mmol) was slowly added dropwise in a cooled state, and potassium thiocyanate (KSCN) (99.3 g, 1022 mmol) and iron chloride (FeCl₃) (36.3 g, 223.6 mmol) were added and stirred at room temperature. After completion of the reaction, the reaction mixture was neutralized with 2M aqueous NaOH solution. The mixture was extracted with chloroform, dried over anhydrous magnesium sulfate, and concentrated, and then purified through a silica column to obtain Compound D-2 (79 g, yield: 69%).

### Step 3: Preparation of Compound D-3

Compound D-2 (79 g, 220 mmol) was added to tetrahydrofuran and cooled to 0°C. Lithium aluminum hydride (LiAlH₄) (1M in THF) (240 mL, 240 mmol) was slowly added dropwise and stirred. When the reaction was completed, water was slowly added thereto and stirred. Then, 2M aqueous HCI solution was added thereto and extracted with ethyl acetate. The separated organic layer was dried over anhydrous magnesium sulfate and concentrated under reduced pressure to obtain Compound D-3 (64 g, yield: 87%).

### Step 4: Preparation of Compound D

Compound D-3 (64 g, 191 mmol) was added to an acetonitrile solvent, cesium carbonate (93.7 g, 287.6 mmol) was added and stirred at 130°C by microwave irradiation. When the reaction was completed, the mixture was washed twice with water and extracted with chloroform. The organic layer was stirred with anhydrous magnesium sulfate, filtered and concentrated under reduced pressure. The concentrated compound was purified through a silica column to prepare Intermediate Compound D (47 g, yield: 83%).
MS:[M+H]⁺ = 296

### [Example]

### Example 1: Preparation of Compound 1-1

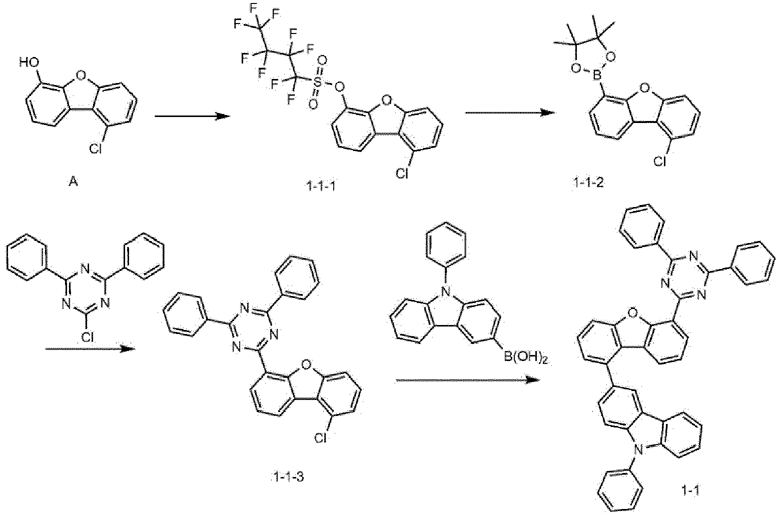

### Step 1) Preparation of Compound 1-1-1

Compound A (21 g, 95 mmol) was dispersed in 200 mL of acetonitrile, and potassium carbonate (26.3 g, 190 mmol) and 40 mL of water were added thereto. Nonafluorobutanesulfonyl fluoride (43 g, 142.5 mmol) was added and the mixture was heated to 80°C. After reacting for 6 hours, the mixture was cooled to room temperature, and the solvent was removed by concentration under reduced pressure. The concentrated compound was re-dissolved in ethyl acetate and washed once with water. The organic layer was separated, treated with anhydrous magnesium sulfate, filtered and then concentrated to give Compound 1-1-1 (35.1 g, yield: 73%).

### Step 2) Preparation of Compound 1-1-2

Compound 1-1-1 (27 g, 54 mmol), bis(pinacolato) diborone (16.4 g, 64.7 mmol) and potassium acetate (10.5 g, 107 mmol) were added to 150 mL of 1,4-dioxane. Dibenzylideneacetone palladium (0.9 g, 3 mol%) and tricyclohexylphosphine (0.9 g, 6 mol%) were added and stirred under reflux for 12 hours. After completion of the reaction, the mixture was cooled to room temperature and filtered through Celite. The filtrate was concentrated under reduced pressure. The residue was dissolved in chloroform, and washed with water to separate the organic layer, followed by drying over anhydrous magnesium sulfate. The resulting material was filtered, distilled under reduced pressure, and stirred with ethyl acetate and ethanol to prepare Compound 1-1-2 (15.2 g, yield: 86%).

### Step 3) Preparation of Compound 1-1-3

Compound 1-1-2 (25 g, 76 mmol) and chloro-4,6-diphenyl-1,3,5-triazine (20.3 g, 76 mmol) was dispersed in 200 mL of tetrahydrofuran. Then, after adding 2M potassium carbonate aqueous solution (aq. K₂CO₃) (57 mL, 114 mmol), tetrakis triphenylphosphine palladium [Pd(PPh₃)₄] (0.88 g, 1 mol%) was added thereto and the mixture was stirred under reflux for 6 hours. The temperature was lowered to room temperature, the water layer was removed, and the organic layer was concentrated under reduced pressure. The concentrated compound was re-dissolved in ethyl acetate and washed twice with water and separated to which anhydrous magnesium sulfate was added, filtered and concentrated. To the concentrated residue was added a small amount of ethyl acetate and an excess amount of hexane, and the mixture was stirred to precipitate a solid, which was stirred for 1 hour and then filtered to obtain Compound 1-1-3 (26.7 g, yield: 81%).

### Step 4) Preparation of Compound 1-1

Compound 1-1-3 (22 g, 50 mmol) and (9-phenyl-9H-carbazol-3-yl)boronic acid (15.2 g, 53 mmol) were dispersed in tetrahydrofuran (200 mL). Then, after adding 2M potassium carbonate aqueous solution (aq. K₂CO₃) (75 mL, 151 mmol), tetrakis triphenylphosphine palladium [Pd(PPh₃)₄] (0.6 g, 1 mol%) was added thereto and the mixture was stirred under reflux for 6 hours. The temperature was lowered to room temperature, the water layer was removed, and the organic layer was concentrated under reduced pressure. Ethyl acetate was added thereto, and the mixture was stirred for 3 hours, and the precipitated solid was filtered. The resulting solid was re-dissolved in chloroform, washed with water, separated, and then treated with anhydrous magnesium sulfate and white acidic clay, followed by filtration. The filtrate was concentrated under reduced pressure to remove about half of the filtrate, and ethyl acetate was added thereto and then recrystallized to prepare Compound 1-1 (26.3 g, yield: 81%).
MS: [M+H]⁺ = 641

### Example 2: Preparation of Compound 1-2

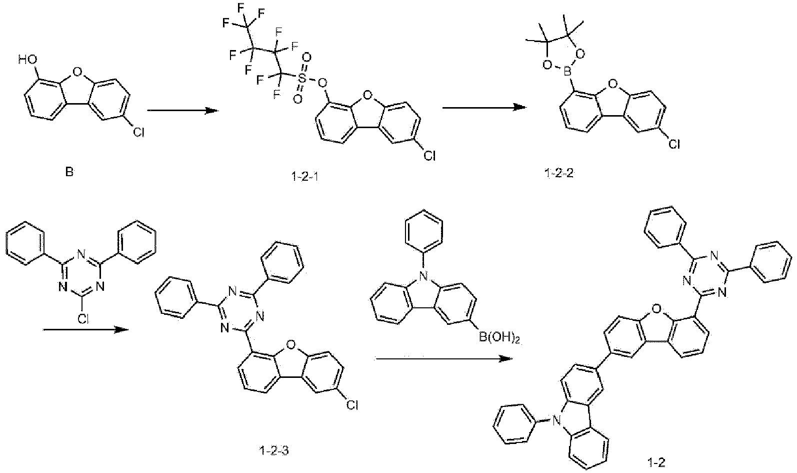

Compound 1-2 was prepared in the same manner as in Preparation Example of Compound 1-1 by using Compound B.
MS: [M+H]⁺ = 641

### Example 3: Preparation of Compound 1-3

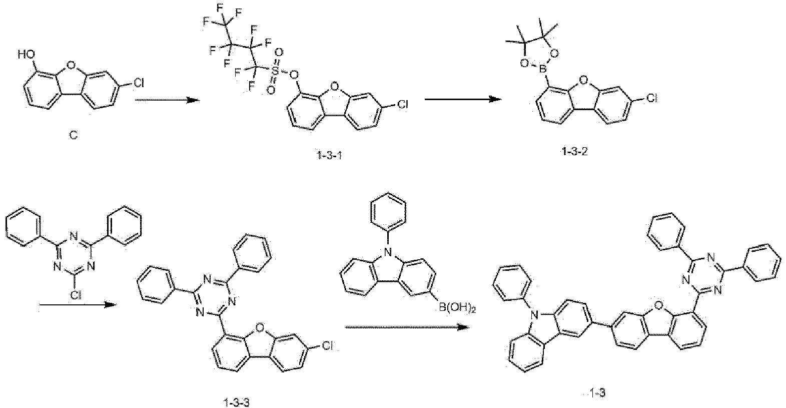

Compound 1-3 was prepared in the same manner as in Preparation Example of Compound 1-1 by using Compound C.
MS:[M+H]⁺=641

### Example 4: Preparation of Compound 1-4

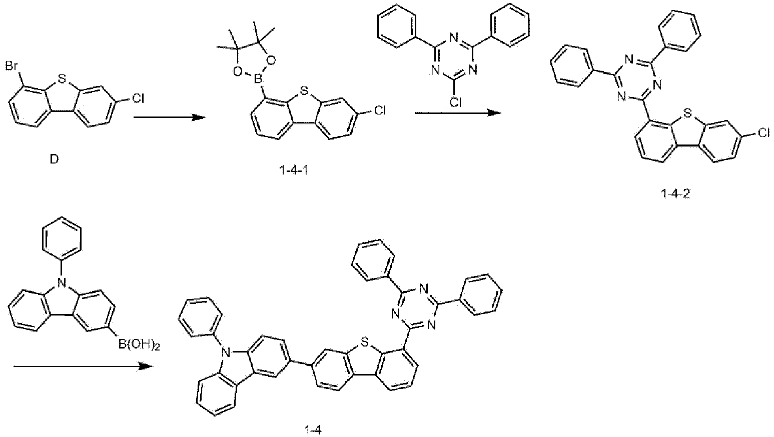

Compound 1-4 was prepared in the same manner as in Preparation Example of Compound 1-1 except for Step 1 of Example 1 by using Compound D.
MS:[M+H]⁺=657

### Example 5: Preparation of Compound 2-1

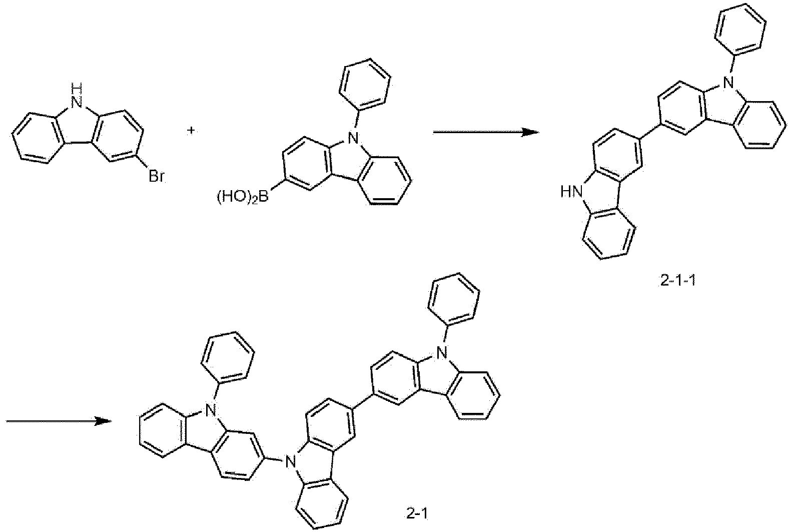

### Step 1) Preparation of Compound 2-1-1

3-Bromo-9H-carbazole (15 g, 61 mmol) and (9-phenyl-9H-carbazole-3-yl)boronic acid (18.4 g, 64 mmol) were dispersed in 150 mL of tetrahydrofuran. Then, after adding 2M potassium carbonate aqueous solution (aq. K₂CO₃) (60 mL, 120 mmol), tetrakis triphenylphosphine palladium [Pd(PPh₃)₄] (1.03 g, 1 mol%) was added thereto and the mixture was stirred under reflux for 8 hours. When the reaction was completed, the temperature was lowered to room temperature, the water layer was removed, and the organic layer was concentrated under reduced pressure, to which ethyl acetate was added and stirred. The resulting solid was filtered to obtain Compound 2-1-1 (30 g, yield: 82%).

### Step 2) Preparation of Compound 2-1

Compound 2-1-1 (12 g, 30 mmol) and 2-bromo-9-phenyl-9H-carbazole (9.5 g, 30 mmol) were dissolved in 150 mL of toluene, and sodium tert-butoxide (5.6 g, 59 mmol) was added thereto and heated. Bis(tri-tert-butylphosphine)palladium (0.15 g, 1 mol%) was added thereto, and stirred under reflux for 12 hours. After completion of the reaction, the temperature was lowered to room temperature, and the resulting solid was filtered. The pale yellow solid was dissolved in chloroform and washed twice with water. The organic layer was separated, and anhydrous magnesium sulfate and a white acidic clay were added thereto, stirred, filtered and concentrated under reduced pressure. The concentrated compound was recrystallized from chloroform and ethyl acetate to obtain Compound 2-1 (14.5 g, yield 76%) as a white solid.

### Example 6: Preparation of Compound 2-2

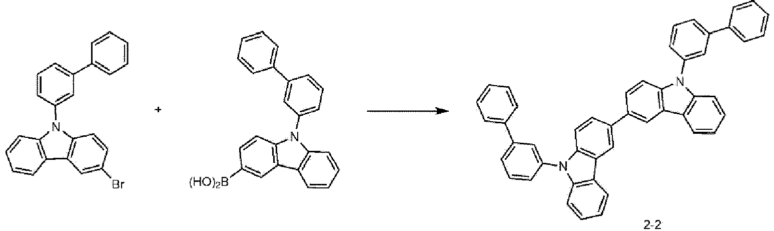

Compound 2-2 (19.7 g, yield: 77%) was prepared in the same manner as in Preparation Example of Compound 2-1-1 by using 9-([1,1'-biphenyl]-3-yl)-3-bromo-carbazole (16 g, 40 mmol) and 9-([1,1'-biphenyl]-3-yl)-9H-carbazole-3-yl)boronic acid (14.6 g, 40 mmol).
MS: [M+H]⁺ = 637

### Example 7: Preparation of Compound 2-3

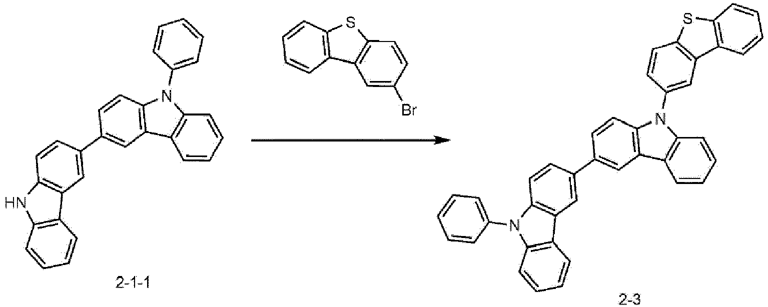

Compound 2-3 (23.1 g, yield: 80%) was prepared in the same manner as in Preparation Example of Compound 2-1 by using Compound 2-1-1 (20 g, 49 mmol) and 1-bromo[b,d]thiophene (12.9 g, 49 mmol).
MS: [M+H]⁺ = 591

### [Experimental Example]

### Experimental Example 1

A glass substrate on which a thin film of ITO (indium tin oxide) was coated in a thickness of 1,300 Å was put into distilled water containing the detergent dissolved therein and washed by the ultrasonic wave. The used detergent was a product commercially available from Fisher Co. and the distilled water was one which had been twice filtered by using a filter commercially available from Millipore Co. The ITO was washed for 30 minutes, and ultrasonic washing was then repeated twice for 10 minutes by using distilled water. After the washing with distilled water was completed, the substrate was ultrasonically washed with isopropyl alcohol, acetone, and methanol solvent, and dried, after which it was transported to a plasma cleaner. Then, the substrate was cleaned with oxygen plasma for 5 minutes, and then transferred to a vacuum evaporator.

On the ITO transparent electrode thus prepared, a compound HI-1 described below was thermally vacuum-deposited in a thickness of 50 Å to form a hole injection layer. A compound HT-1 was thermally vacuum-deposited on the hole injection layer in a thickness of 250 Å to form a hole transport layer. A compound HT-2 was vacuum deposited on the HT-1 vapor deposition layer in a thickness of 50 Å to form an electron blocking layer. Then, the compound 1-1 previously prepared and the compound 2-1 previously prepared were deposited by co-evaporation at a weight ratio shown in Table 1 below, wherein the following compound YGD as a phosphorescent dopant was co-deposited at the weight ratio shown in Table 1 (12%: relative to the total weight of Compound 1-1, Compound 2-1, and YGD) to form a light emitting layer having a thickness (400 Å) in Table 1 below. The following compound ET-1 was vacuum deposited on the light emitting layer in a thickness of 250 Å, and further the compound ET-2 was co-deposited with 2 wt% Li in a thickness of 100 Å to form an electron transport layer and an electron injection layer. Aluminum was deposited on the electron injection layer in a thickness of 1000 Å to form a cathode.

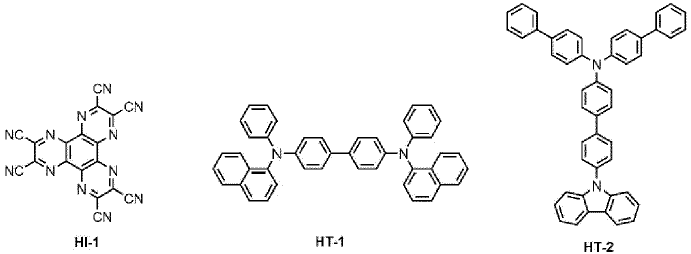

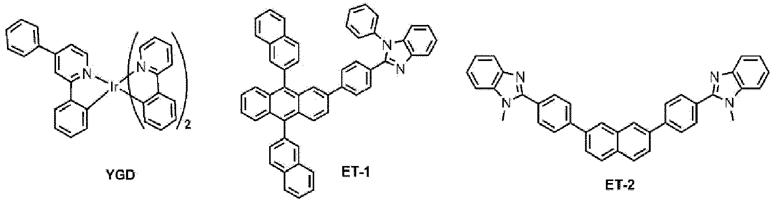

In the above process, the vapor deposition rate of the organic material was maintained at 0.4 to 0.7 Å/sec, the deposition rate of aluminum was maintained at 2 Å /sec, and the degree of vacuum during vapor deposition was maintained at 1×10⁻⁷∼5×10⁻⁸ torr.

### Experimental Examples 2 to 7

The organic light emitting devices were manufactured in the same manner as in Experimental Example 1, except that the contents of the phosphorescent host material and the dopant were changed as shown in Table 1 below, respectively.

### Comparative Experimental Examples 1 to 10

The organic light emitting devices were manufactured in the same manner as in Experimental Example 1, except that the contents of the phosphorescent host material and the dopant were changed as shown in Table 1 below, respectively.

At this time, the host compounds A to C used are as follows.

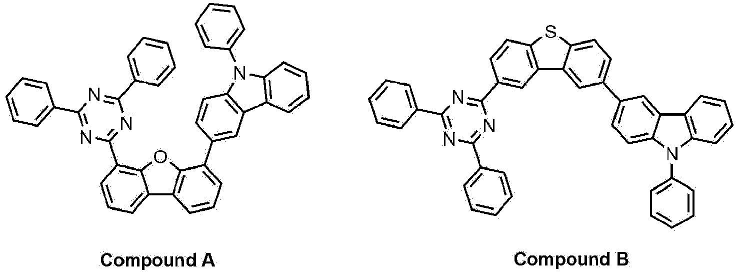

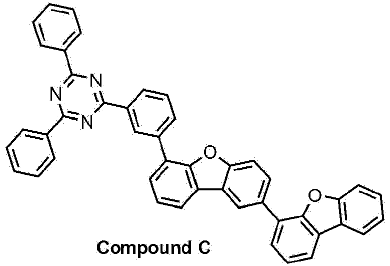

The voltage, efficiency, luminance, color coordinate, and lifetime were measured by applying a current to the organic light emitting devices manufactured in the Experimental Examples and Comparative Experimental Examples, and the results are shown in Table 1 below. At this time, T95 means the time required for the luminance to be reduced to 95% when the initial luminance at the current density of 50 mA/cm² is taken as 100%.

**[Table 1]**

| | | Voltage (V) (@10mA/cm²) | Efficiency (Cd/A) (@10mA/cm²) | Color Coordinate (x,y) | Lifetime (T95, h) (@50mA/cm²) |
|---|---|---|---|---|---|
| Experimental Ex. 1 | (Compound 1-1:Compound2-1)/YGD (200:200)/15% | 3.73 | 23.8 | (0.470,0.522) | 138 |
| Experimental Ex. 2 | (Compound 1-1:Compound 2-3)/YGD (120:280)/12% | 3.37 | 22.8 | (0.469,0.523) | 150 |
| Experimental Ex. 3 | (Compound 1-2:Compound 2-1)/YGD (200:200)/15% | 3.58 | 24.0 | (0.464,0.527) | 118 |
| Experimental Ex. 4 | (Compound 1-2:Compound 2-3)/YGD (280:120)/12% | 3.78 | 24.7 | (0.455,0.537) | 131 |
| Experimental Ex. 5 | (Compound 1-3:Compound 2-1)/YGD (200:200)/12% | 3.69 | 24.4 | (0.460,0.532) | 134 |
| Experimental Ex. 6 | (Compound 1-3:Compound 2-2)/YGD (280:120)/12% | 3.49 | 24.0 | (0.462,0.529) | 90 |
| Experimental Ex. 7 | (Compound 1-4:Compound 2-1)/YGD (200:200)/12% | 3.59 | 23.6 | (0.464,0.527) | 143 |
| Comparative Experimental Ex. 1 | (Compound 1-1)/YGD (400)/12% | 3.00 | 21.7 | (0.464,0.527) | 40 |
| Comparative Experimental Ex. 2 | (Compound 1-4)/YGD (400)/12% | 3.45 | 22.6 | (0.465,0.527) | 33 |
| Comparative Experimental Ex. 3 | (Compound 2-1)/YGD (400)/12% | 5.17 | 4.3 | (0.433,0.528) | 27 |
| Comparative Experimental Ex. 4 | (Compound A)/YGD (400)/12% | 3.17 | 21.2 | (0.472,0.520) | 29 |
| Comparative Experimental Ex. 5 | (Compound B)/YGD (400)/12% | 3.10 | 21.9 | (0.467,0.525) | 25 |
| Comparative Experimental Ex. 6 | (Compound C)/YGD (400)/12% | 3.67 | 21.3 | (0.478,0.516) | 20 |
| Comparative Experimental Ex. 7 | (Compound A:Compound2-1)/YGD (200/200)/12% | 3.92 | 22.3 | (0.461,0.530) | 77 |
| Comparative Experimental Ex. 8 | (Compound A:Compound2-3)/YGD (120:280)/12% | 3.97 | 23.6 | (0.453,0.537) | 59 |
| Comparative Experimental Ex. 9 | (Compound B:Compound 2-2)/YGD (200/200)/12% | 3.78 | 25.1 | (0.448,0.542) | 32 |
| Comparative Experimental Ex.10 | (Compound C:Compound 2-1)/YGD (200:200)/12% | 3.54 | 23.6 | (0.454,0.537) | 37 |

### [Explanation of Sign]

| | |
|---|---|
| 1: substrate, | 2: anode, |
| 3: light emitting layer | 4: cathode |
| 5: hole injection layer | 6: hole transport layer |
| 7: light emitting layer | 8: electron transport layer |

## Claims

1. An organic light emitting device comprising:
an anode; a cathode; and at least one light emitting layer interposed between the anode and the cathode,
wherein the light emitting layer comprises a first host compound represented by Chemical Formulas 1-1, 1-2, or 1-3 below, and a second host compound represented by Chemical Formula 2 below: 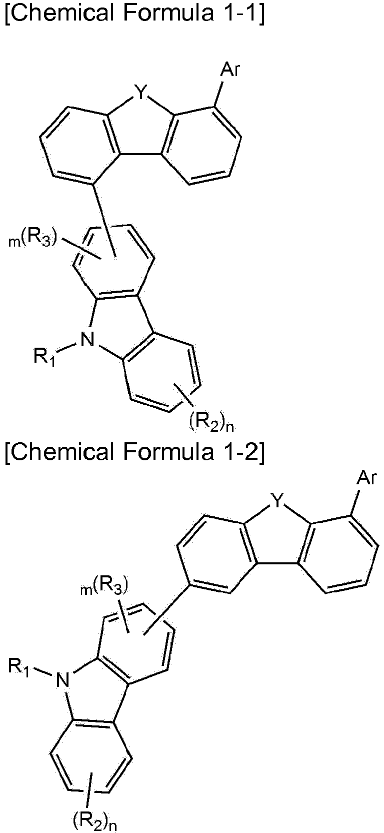 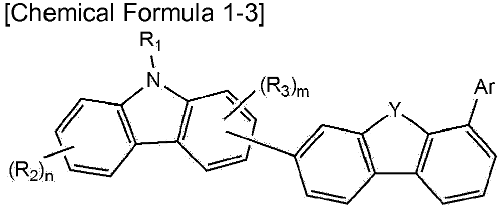 in Chemical Formulas 1-1, 1-2, or 1-3,
R₁ is a substituted or unsubstituted C₁₋₆₀ alkyl; a substituted or unsubstituted C₃₋₆₀ cycloalkyl; a substituted or unsubstituted C₆₋₆₀ aryl; or a substituted or unsubstituted C₂₋₆₀ heteroaryl containing at least one of O, N, Si and S,
R₂ and R₃ are each independently hydrogen; deuterium; halogen; cyano; a substituted or unsubstituted C₁₋₆₀ alkyl; or a substituted or unsubstituted C₃₋₆₀ cycloalkyl,
n is an integer of 0 to 4,
m is an integer of 0 to 3,
Y is O, S, or CR₄R₅,
R₄ and R₅ are each independently hydrogen; deuterium; halogen; cyano; a substituted or unsubstituted C₁₋₆₀ alkyl; a substituted or unsubstituted C₃₋₆₀ cycloalkyl; or a substituted or unsubstituted C₆₋₆₀ aryl,
Ar is represented by the following Chemical Formula 1' 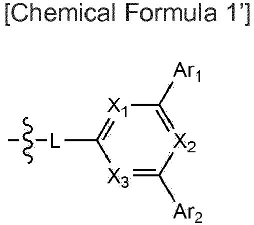
wherein,
L is a single bond; a substituted or unsubstituted C₆₋₆₀ arylene; or a substituted or unsubstituted C₂₋₆₀ heteroarylene containing at least one of O, N, Si and S,
X₁ to X₃ are each independently N, or CR₆, provided that at least one of X₁ to X₃ is N,
each R₆ is independently hydrogen; deuterium; halogen; cyano; nitro; amino; a substituted or unsubstituted C₁₋₆₀ alkyl; a substituted or unsubstituted C₁₋₆₀ haloalkyl; a substituted or unsubstituted C₁₋₆₀ haloalkoxy; a substituted or unsubstituted C₃₋₆₀ cycloalkyl; a substituted or unsubstituted C₂₋₆₀ alkenyl; a substituted or unsubstituted C₆₋₆₀ aryl; or a substituted or unsubstituted C₂₋₆₀ heteroaryl containing at least one of O, N, Si and S,
Ar₁ and Ar₂ are each independently a substituted or unsubstituted C₆₋₆₀ aryl; or a substituted or unsubstituted C₂₋₆₀ heteroaryl containing at least one of O, N, Si and S, 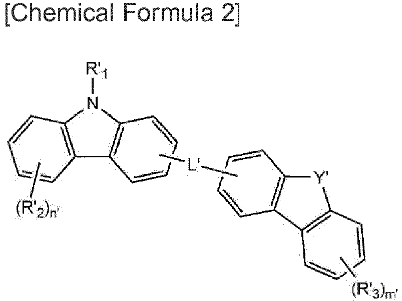
in Chemical Formula 2,
R'₁ is cyclohexyl, phenyl, cyano-substituted phenyl, biphenylyl, terphenylyl, naphthyl, phenanthryl, triphenylenyl, dimethylfluorenyl, pyridinyl, dibenzofuranyl, dibenzothiophenyl, or 9-phenyl-carbazolyl,
R'₂ and R'₃ are each independently hydrogen; deuterium; halogen; cyano; a substituted or unsubstituted C₁₋₆₀ alkyl; a substituted or unsubstituted C₃₋₆₀ cycloalkyl; a substituted or unsubstituted C₆₋₆₀ aryl; or a substituted or unsubstituted C₂₋₆₀ heteroaryl containing at least one of O, N, Si and S,
n' and m' are each independently an integer of 0 to 4,
L' is a single bond; or a substituted or unsubstituted C₆₋₆₀ arylene,
Y' is O, S, NR', or CR'R",
R' and R" are each independently a substituted or unsubstituted C₁₋₆₀ alkyl; a substituted or unsubstituted C₃₋₆₀ cycloalkyl; or a substituted or unsubstituted C₆₋₆₀ aryl;, or R' and R" together form a substituted or unsubstituted C₆₋₆₀ aromatic ring,
and wherein the first host compound and the second host compound are different from each other.

2. The organic light emitting device of claim 1, wherein
R₁ is phenyl; or biphenylyl.

3. The organic light emitting device of claim 1, wherein
R₂ and R₃ are hydrogen.

4. The organic light emitting device of claim 1, wherein
Y is S, or O.

5. The organic light emitting device of claim 1, wherein
L is a single bond, or phenylene.

6. The organic light emitting device of claim 1, wherein
Ar₁ and Ar₂ are each independently phenyl, cyano-substituted phenyl, biphenylyl, dimethylfluorenyl, naphthyl, phenanthryl, pyridinyl, dibenzofuranyl, or dibenzothiophenyl.

7. The organic light emitting device of claim 1, wherein
the compound represented by Chemical Formulas 1-1, 1-2, or 1-3 is any one selected from the group consisting of the following:

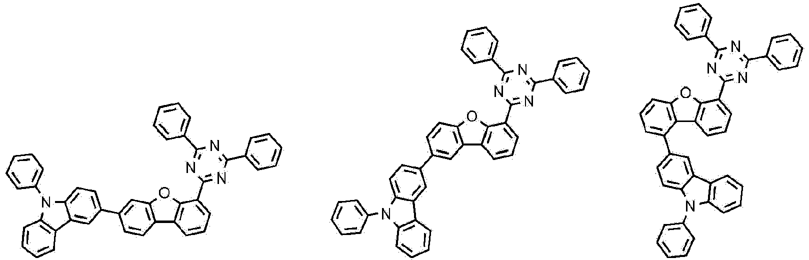

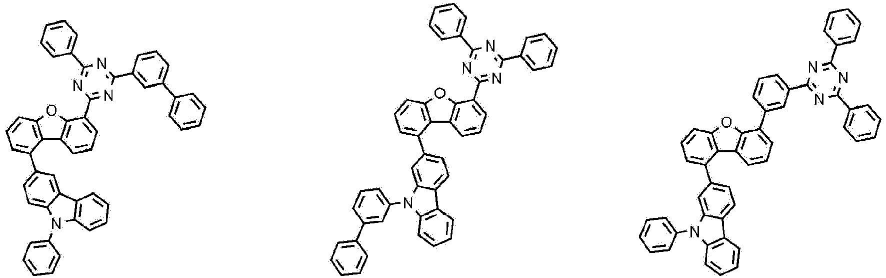

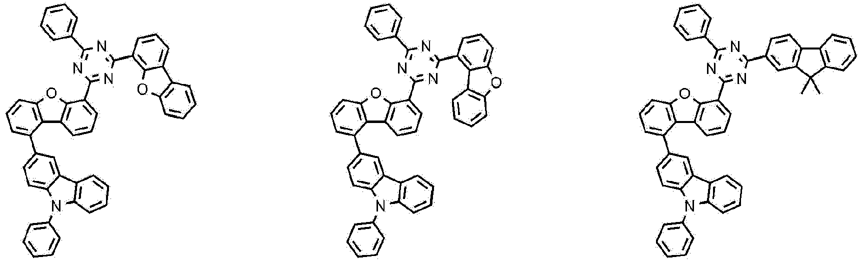

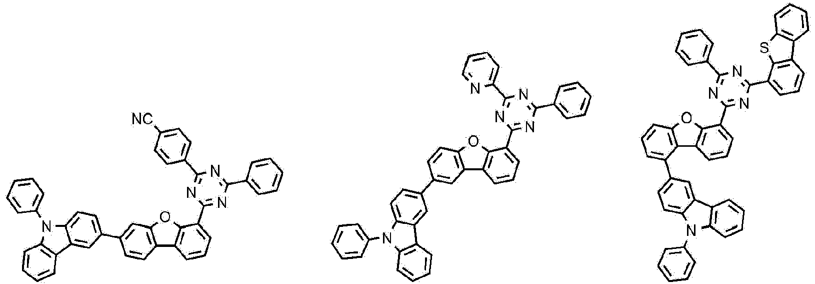

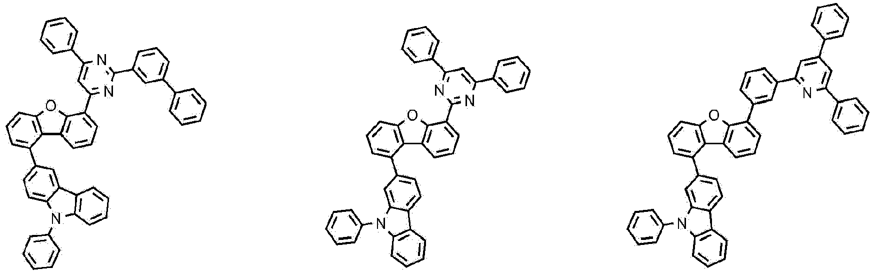

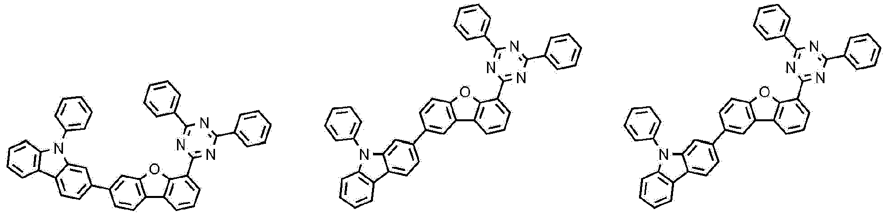

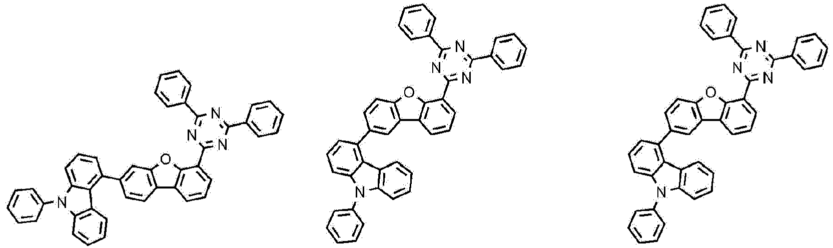

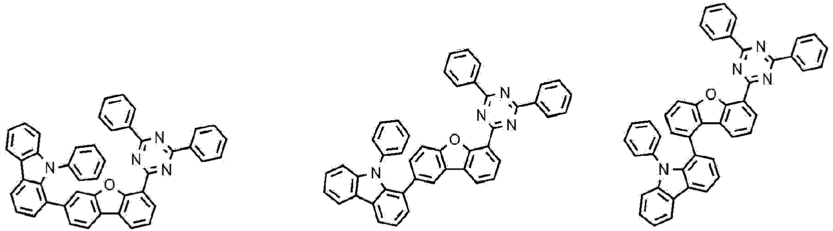

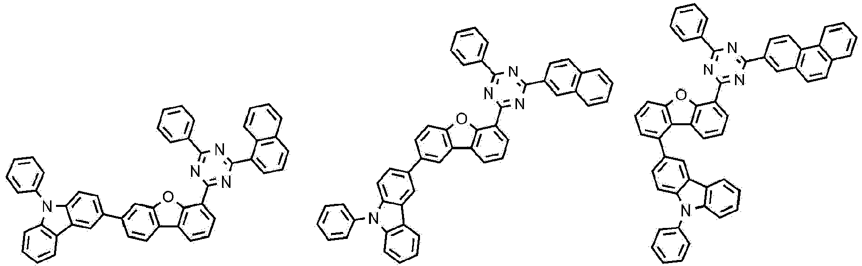

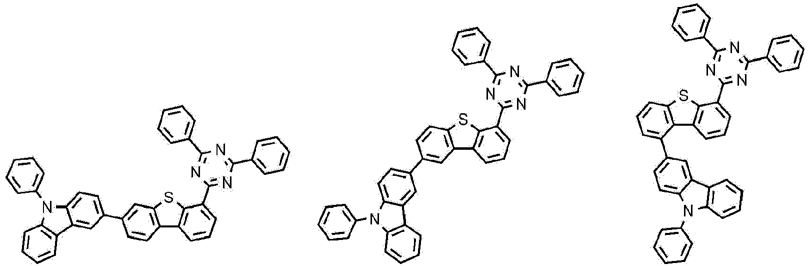

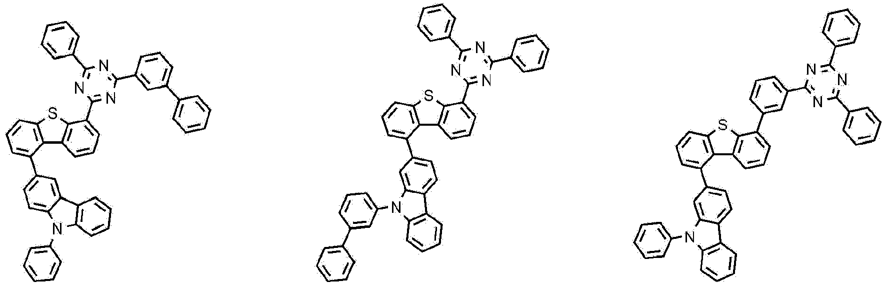

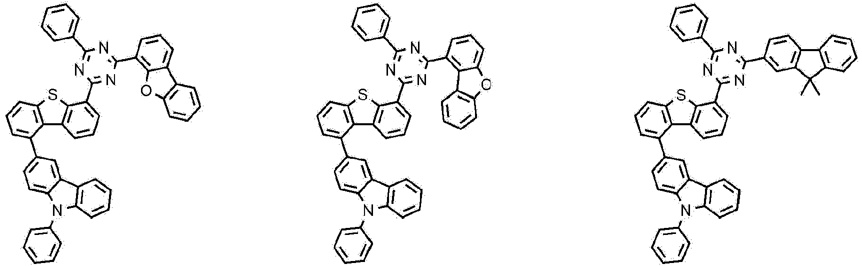

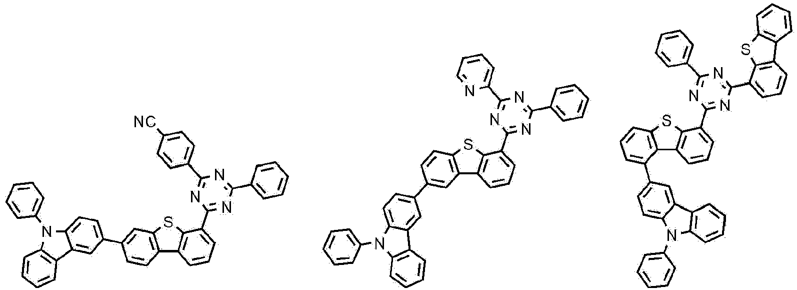

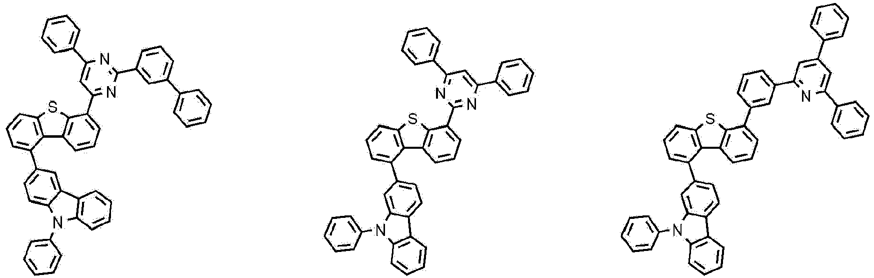

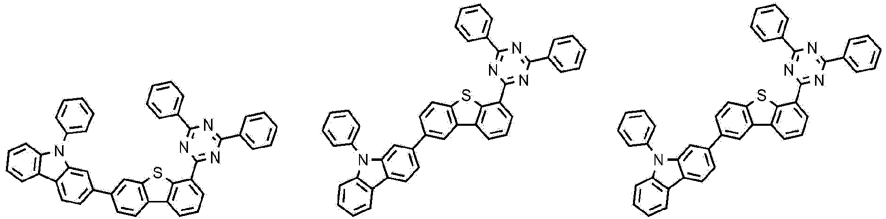

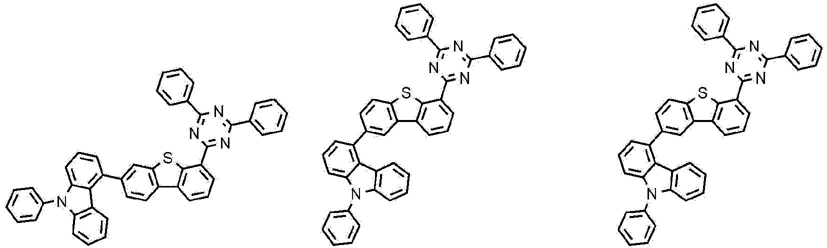

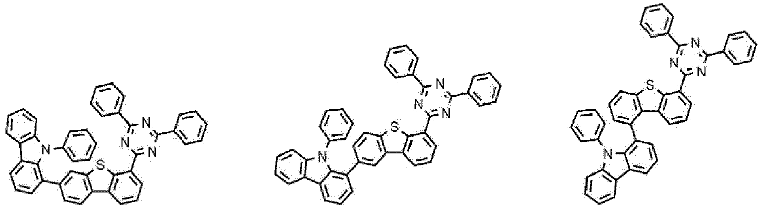

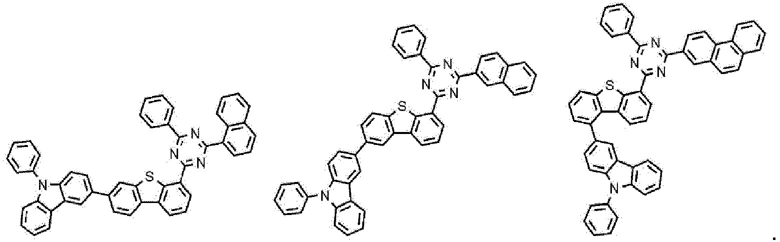

8. The organic light emitting device of claim 1, wherein
n' is 1, and
R'₂ is hydrogen, or phenyl.

9. The organic light emitting device of claim 1, wherein
m' is 1, and
R'₃ is hydrogen, tert-butyl, cyano, phenyl, cyano-substituted phenyl, or pyridinyl.

10. The organic light emitting device of claim 1, wherein
Y' is O, S, or NR', and
R' is phenyl, or biphenylyl.

11. The organic light emitting device of claim 1, wherein
Y' is CR'R", and
R' and R" are methyl, or R' and R" together form a fluorene ring.

12. The organic light emitting device of claim 1, wherein
L' is a single bond, or phenylene.

13. The organic light emitting device of claim 1, wherein
the compound represented by Chemical Formula 2 is any one selected from the group consisting of the following:

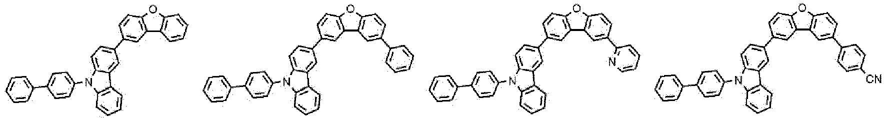

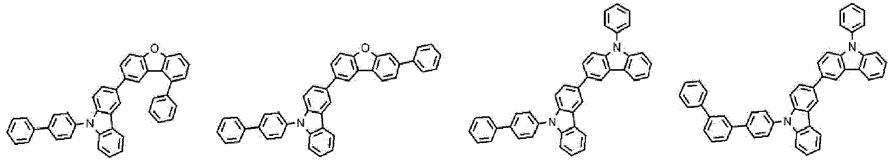

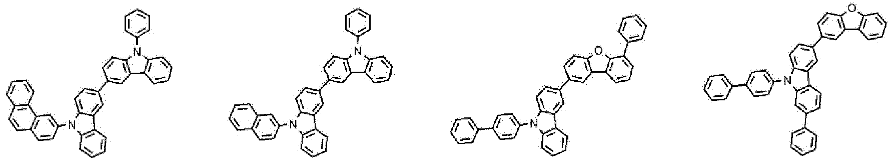

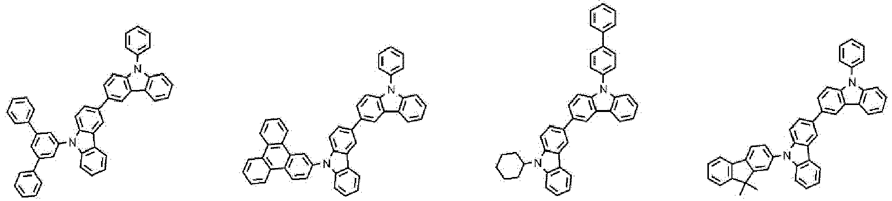

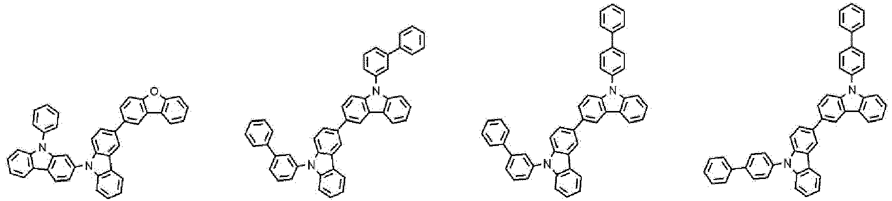

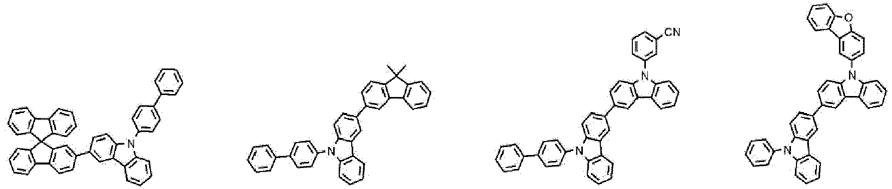

## Patentansprüche

1. Organische lichtemittierende Vorrichtung, umfassend:
eine Anode, eine Kathode und mindestens eine lichtemittierende Schicht, die zwischen der Anode und der Kathode angeordnet ist,
wobei die lichtemittierende Schicht eine erste Wirtsverbindung, die durch die nachstehende chemische Formel 1-1, 1-2 oder 1-3 dargestellt ist, und eine zweite Wirtsverbindung, die durch die nachstehende chemische Formel 2 dargestellt ist, umfasst: 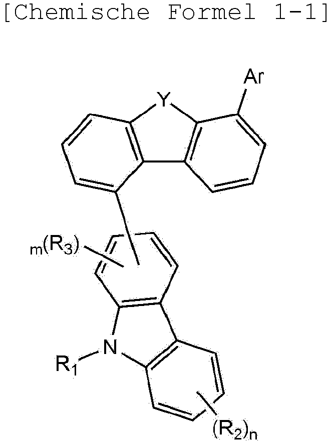 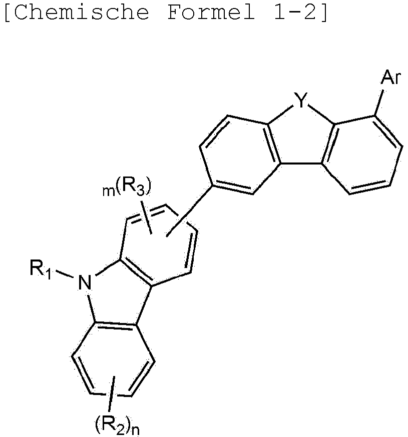 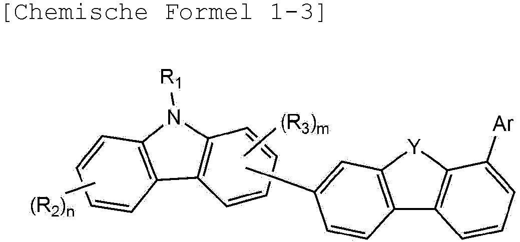 in den chemischen Formeln 1-1, 1-2 oder 1-3
R₁ ein substituiertes oder unsubstituiertes C₁₋₆₀-Alkyl, ein substituiertes oder unsubstituiertes C₃₋₆₀-Cycloalkyl, ein substituiertes oder unsubstituiertes C₆₋₆₀-Aryl oder ein substituiertes oder unsubstituiertes C₂₋₆₀-Heteroaryl, das mindestens eines von O, N, Si und S enthält, ist,
R₂ und R₃ jeweils unabhängig Wasserstoff, Deuterium, Halogen, Cyano, ein substituiertes oder unsubstituiertes C₁₋₆₀-Alkyl oder ein substituiertes oder unsubstituiertes C₃₋₆₀-Cycloalkyl sind,
n eine ganze Zahl von 0 bis 4 ist,
m eine ganze Zahl von 0 bis 3 ist,
Y O, S oder CR₄R₅ ist,
R₄ und R₅ jeweils unabhängig Wasserstoff, Deuterium, Halogen, Cyano, ein substituiertes oder unsubstituiertes C₁₋₆₀-Alkyl, ein substituiertes oder unsubstituiertes C₃₋₆₀-Cycloalkyl oder ein substituiertes oder unsubstituiertes C₆₋₆₀-Aryl sind,
Ar durch die folgende chemische Formel 1' dargestellt ist 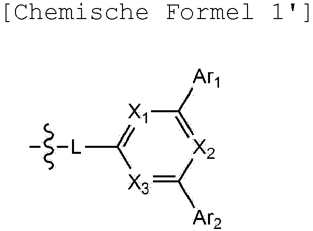
worin
L eine Einfachbindung, ein substituiertes oder unsubstituiertes C₆₋₆₀-Arylen oder ein substituiertes oder unsubstituiertes C₂₋₆₀-Heteroarylen, das mindestens eins von O, N, Si und S enthält, ist,
X₁ bis X₃ jeweils unabhängig N oder CR₆ sind, vorausgesetzt, dass mindestens eines von X₁ bis X₃ N ist,
jedes R₆ unabhängig Wasserstoff, Deuterium, Halogen, Cyano, Nitro, Amino, ein substituiertes oder unsubstituiertes C₁₋₆₀-Alkyl, ein substituiertes oder unsubstituiertes C₁₋₆₀-Halogenalkyl, ein substituiertes oder unsubstituiertes C₁₋₆₀-Halogenalkoxy, ein substituiertes oder unsubstituiertes C₃₋₆₀-Cycloalkyl, ein substituiertes oder unsubstituiertes C₂₋₆₀-Alkenyl, ein substituiertes oder unsubstituiertes C₆₋₆₀-Aryl oder ein substituiertes oder unsubstituiertes C₂₋₆₀-Heteroaryl, das mindestens eines von O, N, Si und S enthält, ist,
Ar₁ und Ar₂ jeweils unabhängig ein substituiertes oder unsubstituiertes C₆₋₆₀-Aryl oder ein substituiertes oder unsubstituiertes C₂₋₆₀-Heteroaryl, das mindestens eines von O, N, Si und S enthält, sind, 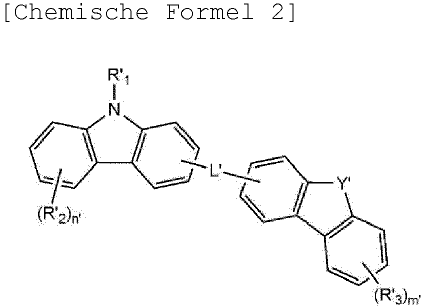
in der chemischen Formel 2
R'₁ Cyclohexyl, Phenyl, Cyano-substituiertes Phenyl, Biphenylyl, Terphenylyl, Naphthyl, Phenanthryl, Triphenylenyl, Dimethylfluorenyl, Pyridinyl, Dibenzofuranyl, Dibenzothiophenyl oder 9-Phenyl-Carbazolyl ist,
R'₂ und R'₃ jeweils unabhängig Wasserstoff, Deuterium, Halogen, Cyano, ein substituiertes oder unsubstituiertes C₁₋₆₀-Alkyl, ein substituiertes oder unsubstituiertes C₃₋₆₀-Cycloalkyl, ein substituiertes oder unsubstituiertes C₆₋₆₀-Aryl oder ein substituiertes oder unsubstituiertes C₂₋₆₀-Heteroaryl, das mindestens eines von O, N, Si und S enthält, sind,
n' und m' jeweils unabhängig eine ganze Zahl von 0 bis 4 sind,
L' eine Einfachbindung oder ein substituiertes oder unsubstituiertes C₆₋₆₀-Arylen ist,
Y' O, S, NR' oder CR'R" ist,
R' und R" jeweils unabhängig ein substituiertes oder unsubstituiertes C₁₋₆₀-Alkyl, ein substituiertes oder unsubstituiertes C₃₋₆₀-Cycloalkyl oder ein substituiertes oder unsubstituiertes C₆₋₆₀-Aryl sind oder R' und R" zusammen einen substituierten oder unsubstituierten aromatischen C₆₋₆₀-Ring bilden,
und wobei die erste Wirtsverbindung und die zweite Wirtsverbindung verschieden voneinander sind.

2. Organische lichtemittierende Vorrichtung gemäß Anspruch 1, wobei
R₁ Phenyl oder Biphenylyl ist.

3. Organische lichtemittierende Vorrichtung gemäß Anspruch 1, wobei
R₂ und R₃ Wasserstoff sind.

4. Organische lichtemittierende Vorrichtung gemäß Anspruch 1, wobei
Y S oder O ist.

5. Organische lichtemittierende Vorrichtung gemäß Anspruch 1, wobei
L eine Einfachbindung oder Phenylen ist.

6. Organische lichtemittierende Vorrichtung gemäß Anspruch 1, wobei
Ar₁ und Ar₂ jeweils unabhängig Phenyl, Cyano-substituiertes Phenyl, Biphenylyl, Dimethylfluorenyl, Naphthyl, Phenanthryl, Pyridinyl, Dibenzofuranyl oder Dibenzothiophenyl sind.

7. Organische lichtemittierende Vorrichtung gemäß Anspruch 1, wobei
die durch die chemischen Formeln 1-1, 1-2 oder 1-3 dargestellte Verbindung irgendeine, ausgewählt aus der Gruppe, bestehend aus den folgenden, ist:

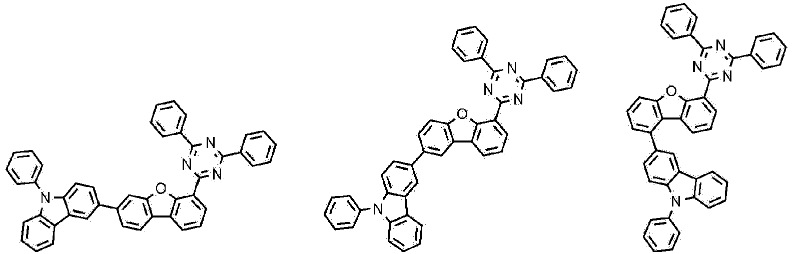

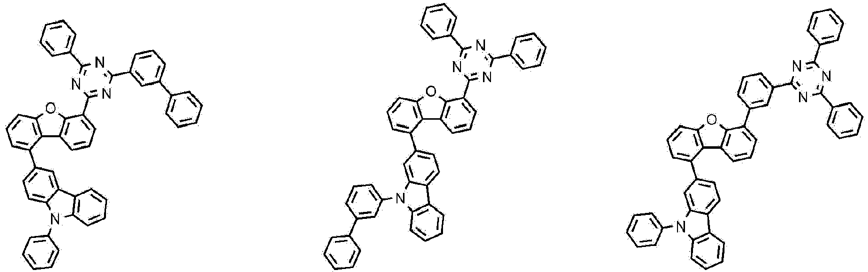

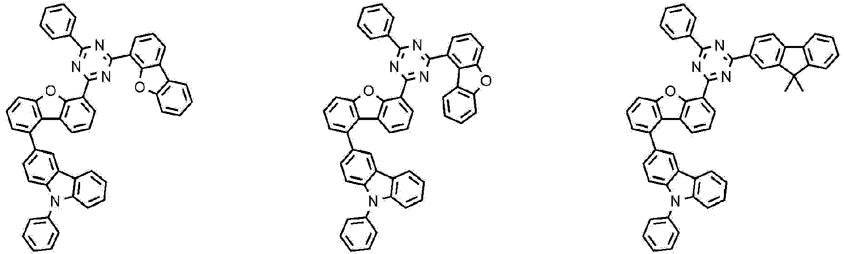

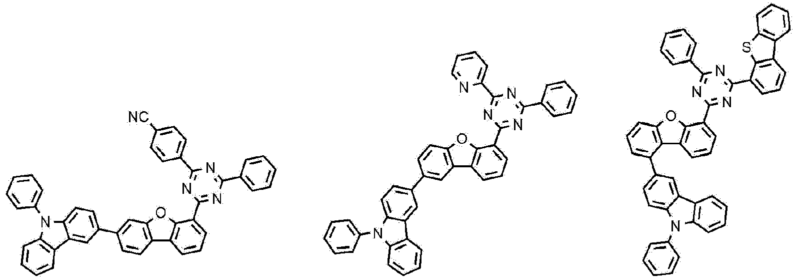

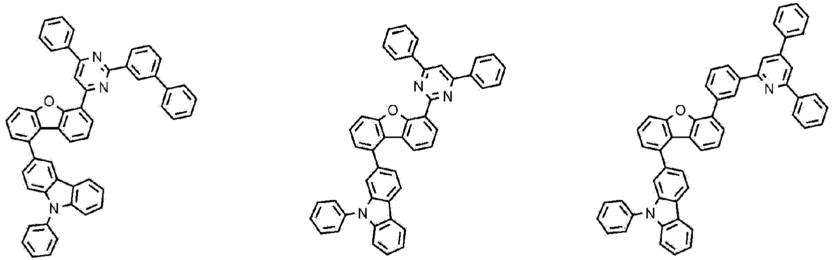

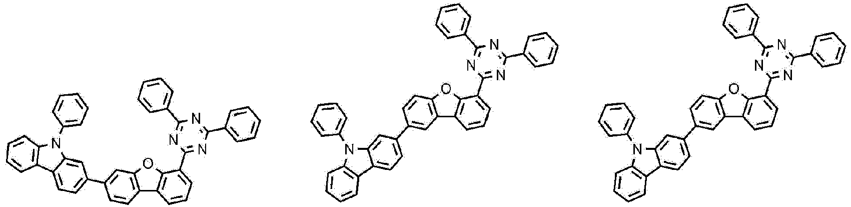

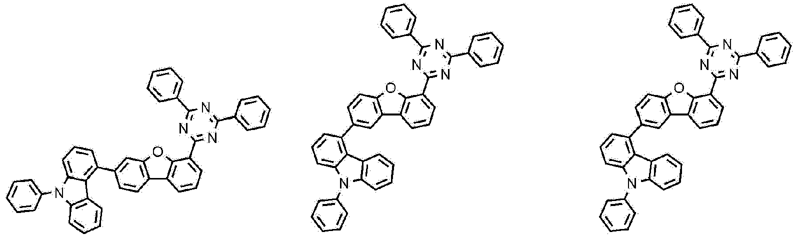

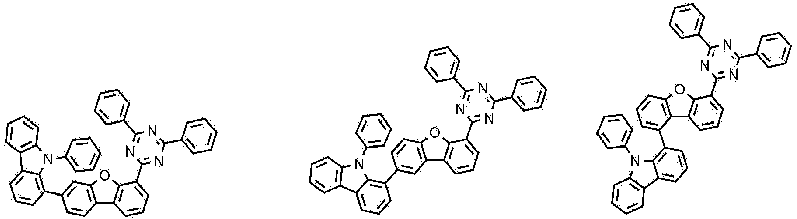

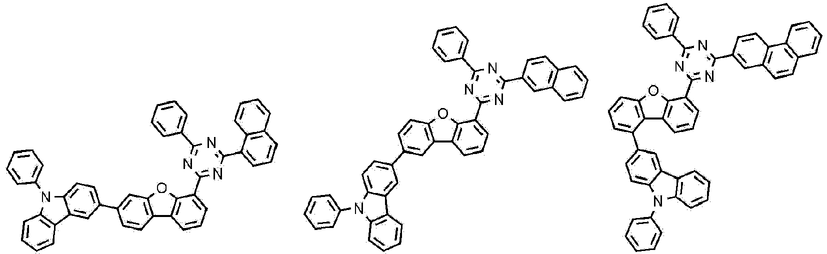

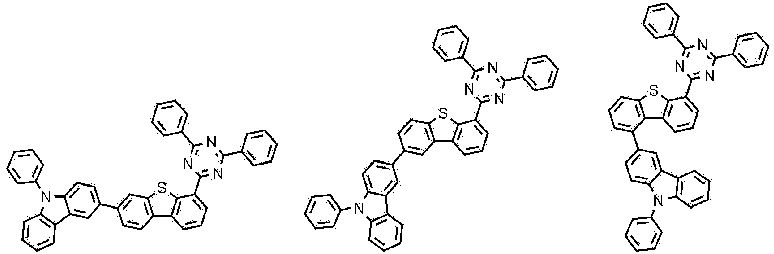

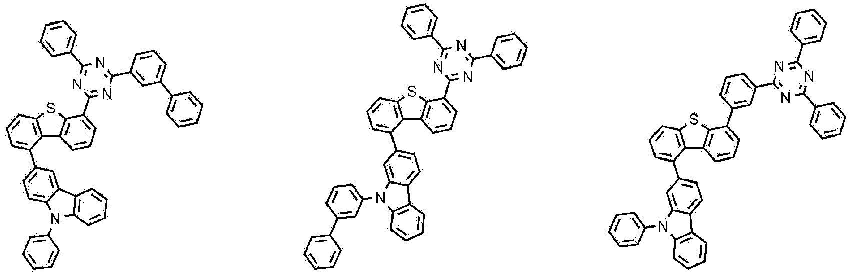

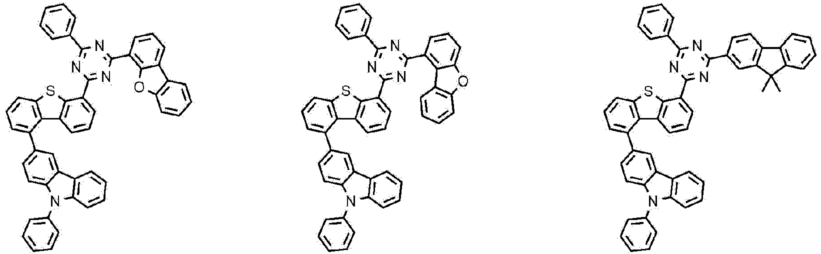

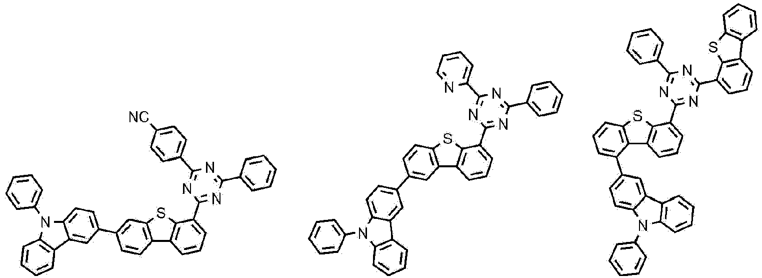

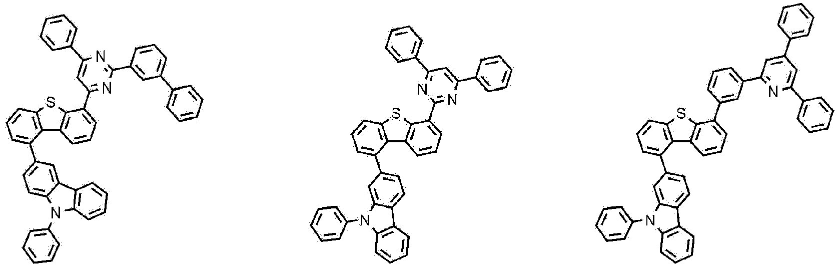

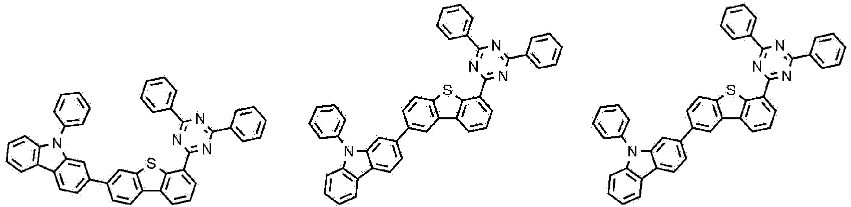

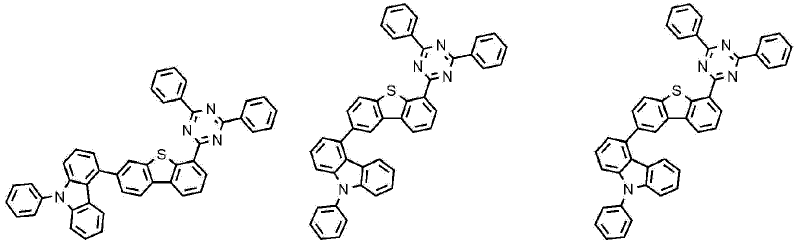

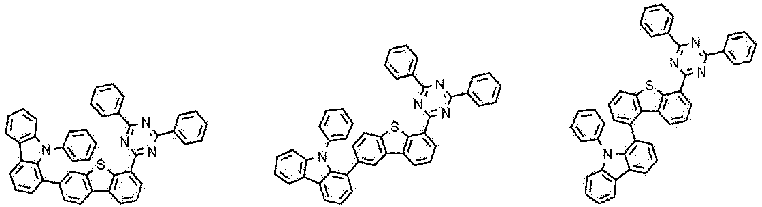

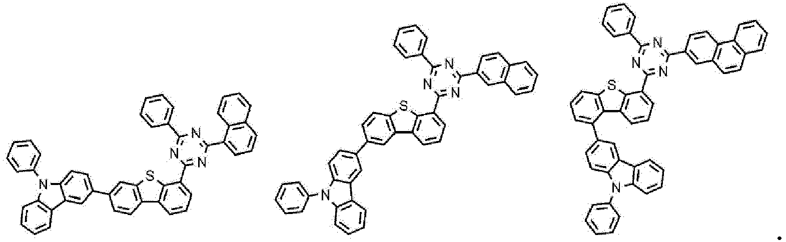

8. Organische lichtemittierende Vorrichtung gemäß Anspruch 1, wobei
n' 1 ist und
R'₂ Wasserstoff oder Phenyl ist.

9. Organische lichtemittierende Vorrichtung gemäß Anspruch 1, wobei
m' 1 ist und
R'₃ Wasserstoff, tert-Butyl, Cyano, Phenyl, Cyano-substituiertes Phenyl oder Pyridinyl ist.

10. Organische lichtemittierende Vorrichtung gemäß Anspruch 1, wobei
Y' O, S oder NR' ist und
R' Phenyl oder Biphenylyl ist.

11. Organische lichtemittierende Vorrichtung gemäß Anspruch 1, wobei
Y' CR'R" ist und
R' und R" Methyl sind oder R' und R" zusammen einen Fluorenring bilden.

12. Organische lichtemittierende Vorrichtung gemäß Anspruch 1, wobei
L' eine Einfachbindung oder Phenylen ist.

13. Organische lichtemittierende Vorrichtung gemäß Anspruch 1, wobei
die durch die chemische Formel 2 dargestellte Verbindung irgendeine, ausgewählt aus der Gruppe, bestehend aus den folgenden, ist:

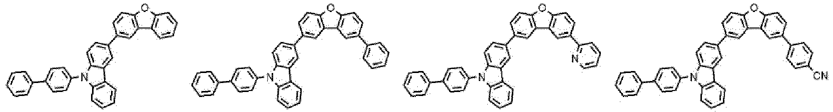

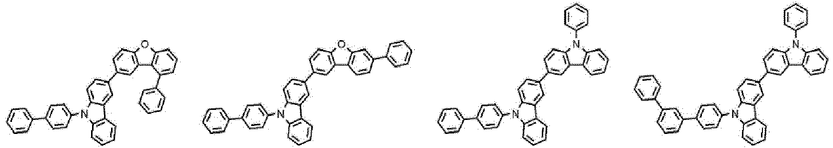

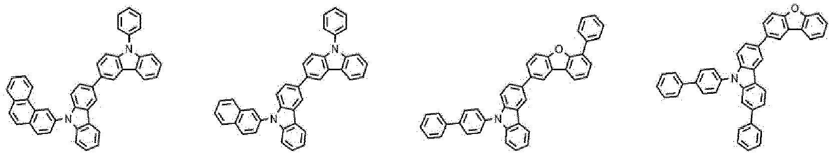

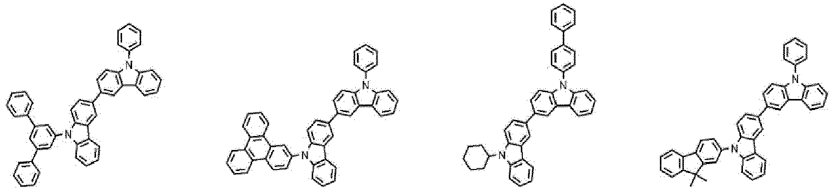

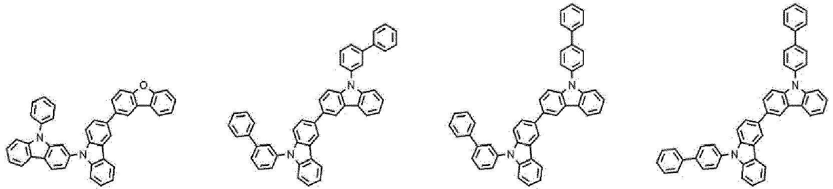

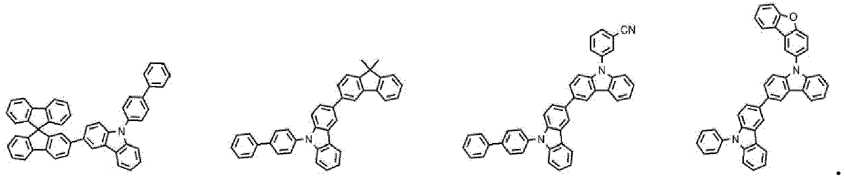

## Revendications

1. Dispositif électroluminescent organique comprenant :
une anode ; une cathode ; et au moins une couche électroluminescente interposée entre l'anode et la cathode,
dans lequel la couche électroluminescente comprend un premier composé hôte représenté par les Formules chimiques 1-1, 1-2 ou 1-3 ci-dessous, et un second composé hôte représenté par la Formule chimique 2 ci-dessous : 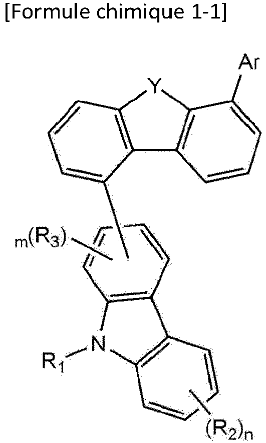 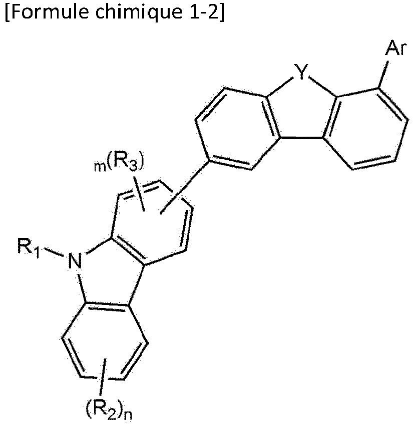 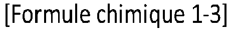 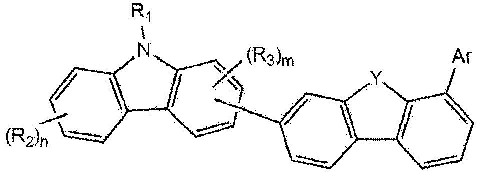 dans les Formules chimiques 1-1, 1-2 ou 1-3,
R₁ est un alkyle en C₁₋₆₀ substitué ou non substitué ; un cycloalkyle en C₃₋₆₀ substitué ou non substitué ; un aryle en C₆₋₆₀ substitué ou non substitué ; ou un hétéroaryle en C₂₋₆₀ substitué ou non substitué contenant au moins un parmi O, N, Si et S,
R₂ et R₃ sont chacun indépendamment un hydrogène ; un deutérium ; un halogène ; un cyano ; un alkyle en C₁₋₆₀ substitué ou non substitué ; ou un cycloalkyle en C₃₋₆₀ substitué ou non substitué,
n est un nombre entier compris entre 0 et 4,
m est un nombre entier compris entre 0 et 3,
Y est O, S, ou CR₄R₅,
R₄ et R₅ sont chacun indépendamment un hydrogène ; un deutérium ; un halogène ; un cyano ; un alkyle en C₁₋₆₀ substitué ou non substitué ; un cycloalkyle en C₃₋₆₀ substitué ou non substitué ; ou un aryle en C₆₋₆₀ substitué ou non substitué,
Ar est représenté par la Formule chimique 1' suivante 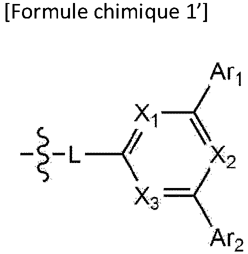
dans laquelle,
L est une liaison simple; un arylène en C₆₋₆₀ substitué ou non substitué; ou un hétéroarylène en C₂₋₆₀ substitué ou non substitué contenant au moins un parmi O, N, Si et S,
X₁ à X₃ sont chacun indépendamment N, ou CR₆, à condition qu'au moins l'un de X₁ à X₃ soit N,
chaque R₆ est indépendamment un hydrogène ; un deutérium ; un halogène ; un cyano ; un nitro ; un amino ; un alkyle en C₁₋₆₀ substitué ou non substitué ; un haloalkyle en C₁₋₆₀ substitué ou non substitué ; un haloalcoxy en C₁₋₆₀ substitué ou non substitué ; un cycloalkyle en C₃₋₆₀ substitué ou non substitué ; un alcényle en C₂₋₆₀ substitué ou non substitué ; un aryle en C₆₋₆₀ substitué ou non substitué ; ou un hétéroaryle en C₂₋₆₀ substitué ou non substitué contenant au moins un parmi O, N, Si et S,
Ar₁ et Ar₂ sont chacun indépendamment un aryle en C₆₋₆₀ substitué ou non substitué ; ou un hétéroaryle en C₂₋₆₀ substitué ou non substitué contenant au moins un parmi O, N, Si et S, 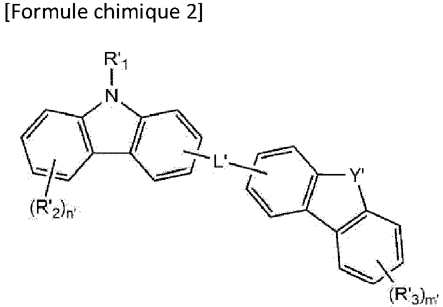
dans la Formule chimique 2,
R'₁ est un cyclohexyle, un phényle, un phényle substitué par un cyano, un biphénylyle, un terphénylyle, un naphtyle, un phénanthryle, un triphénylényle, un diméthylfluorényle, un pyridinyle, un dibenzofuranyle, un dibenzothiophényle, ou un 9-phényl-carbozolyle,
R'₂ et R'₃ sont chacun indépendamment un hydrogène; un deutérium ; un halogène ; un cyano ; un alkyle en C₁₋₆₀ substitué ou non substitué ; un cycloalkyle en C₃₋₆₀ substitué ou non substitué ; un aryle en C₆₋₆₀ substitué ou non substitué ; ou un hétéroaryle en C₂₋₆₀ substitué ou non substitué contenant au moins un parmi O, N, Si et S,
n' et m' sont chacun indépendamment un nombre entier compris entre 0 et 4,
L' est une liaison simple ; ou un arylène en C₆₋₆₀ substitué ou non substitué,
Y' est O, S, NR', ou CR'R",
R' et R" sont chacun indépendamment un alkyle en C₁₋₆₀ substitué ou non substitué ; un cycloalkyle en C₃₋₆₀ substitué ou non substitué ; ou un aryle en C₆₋₆₀ substitué ou non substitué ; ou R' et R" ensemble forment un cycle aromatique en C₆₋₆₀ substitué ou non substitué,
et dans lequel le premier composé hôte et le second composé hôte sont différents l'un de l'autre.

2. Dispositif électroluminescent organique selon la revendication 1, dans lequel
R₁ est un phényle ; ou un biphénylyle.

3. Dispositif électroluminescent organique selon la revendication 1, dans lequel
R₂ et R₃ sont un hydrogène.

4. Dispositif électroluminescent organique selon la revendication 1, dans lequel
Y est S, ou O.

5. Dispositif électroluminescent organique selon la revendication 1, dans lequel
L est une liaison simple, ou un phénylène.

6. Dispositif électroluminescent organique selon la revendication 1, dans lequel
Ar₁ et Ar₂ sont chacun indépendamment un phényle, un phényle substitué par un cyano, un biphénylyle, un diméthylfluorényle, un naphtyle, un phénanthryle, un pyridinyle, un dibenzofuranyle, ou un dibenzothiophényle.

7. Dispositif électroluminescent organique selon la revendication 1, dans lequel
le composé représenté par les Formules chimiques 1-1, 1-2 ou 1-3 est un quelconque sélectionné à partir du groupe consistant en les suivants :

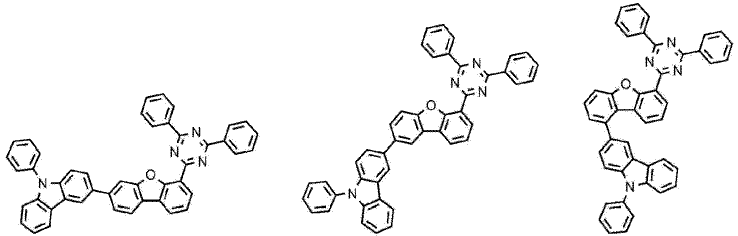

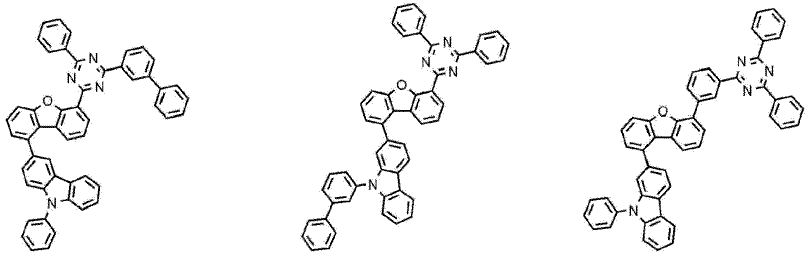

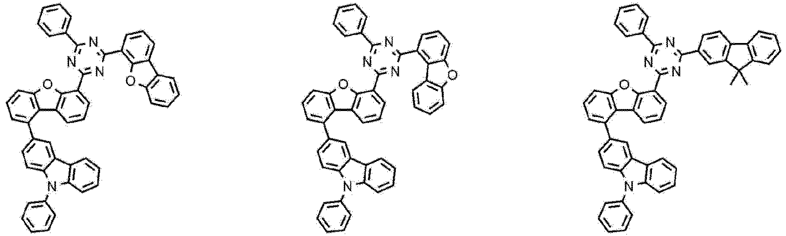

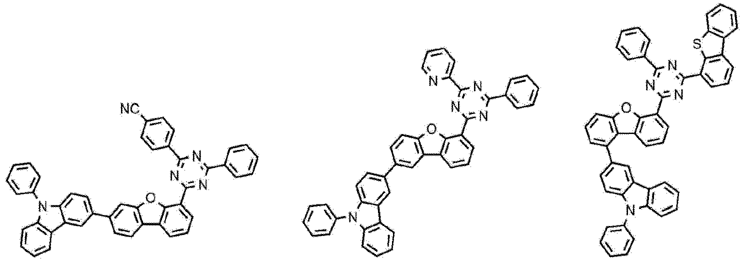

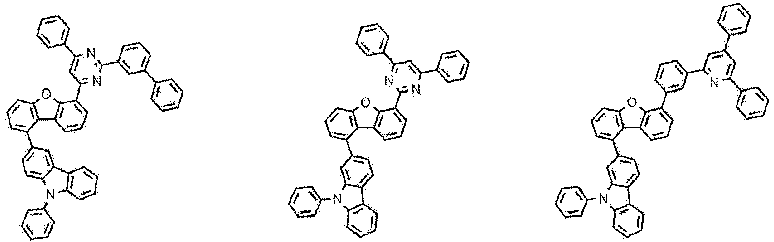

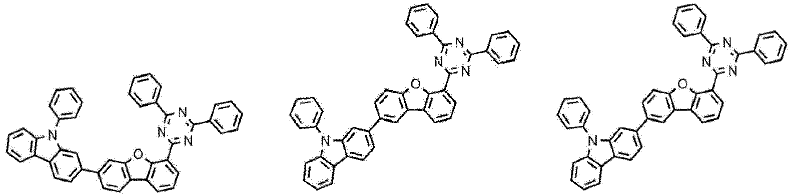

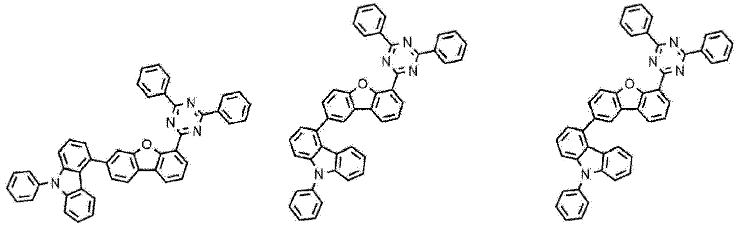

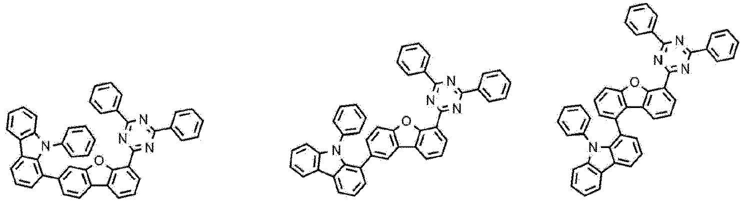

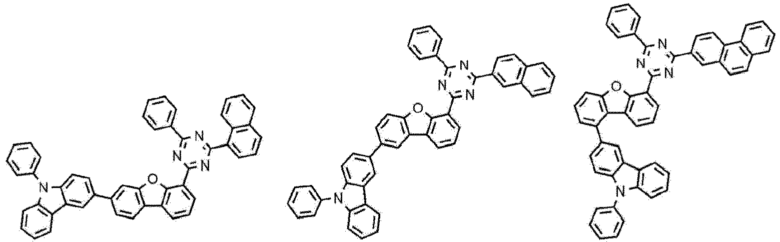

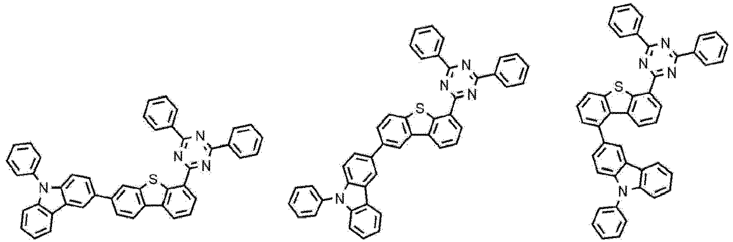

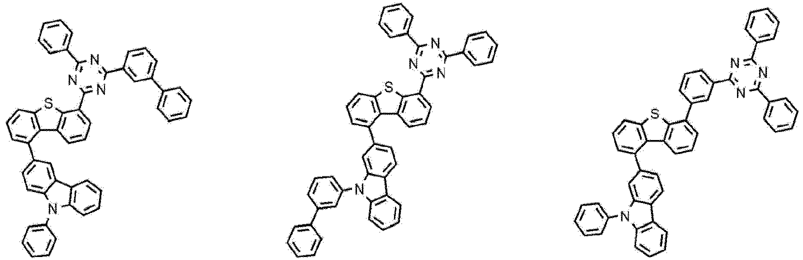

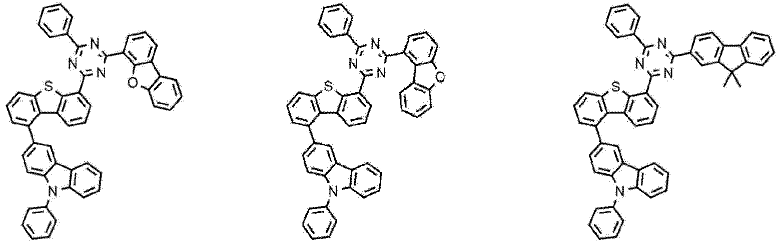

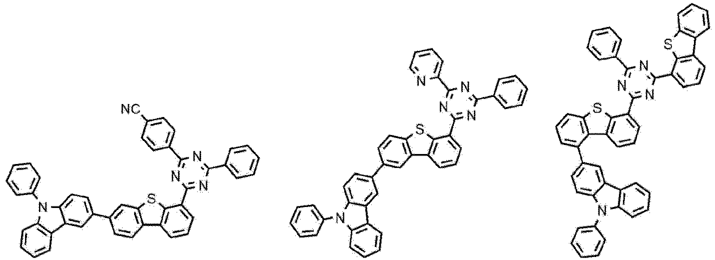

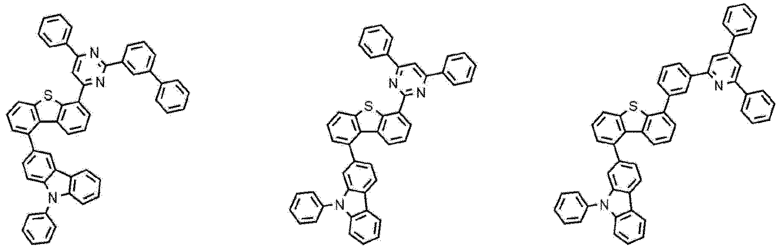

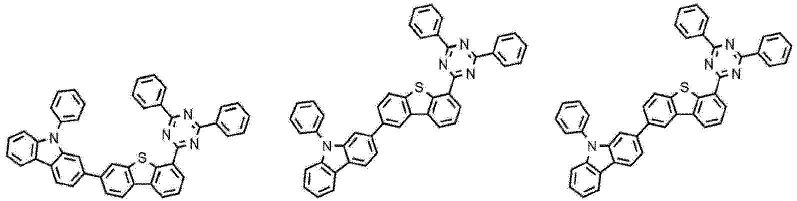

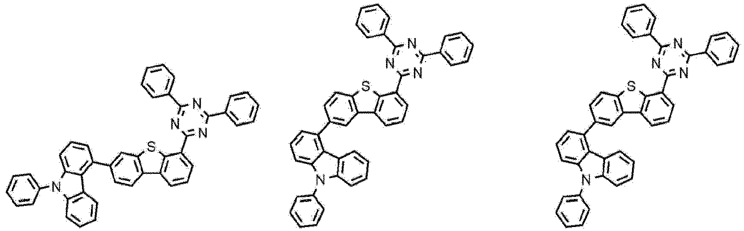

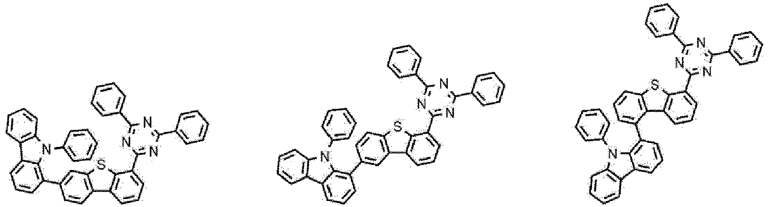

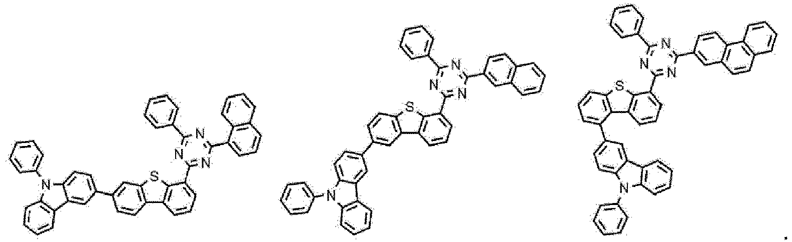

8. Dispositif électroluminescent organique selon la revendication 1, dans lequel
n'est 1, et
R'₂ est un hydrogène, ou un phényle.

9. Dispositif électroluminescent organique selon la revendication 1, dans lequel
m' est 1, et
R'₃ est un hydrogène, un tert-butyle, un cyano, un phényle, un phényle substitué par un cyano, ou un pyridinyle.

10. Dispositif électroluminescent organique selon la revendication 1, dans lequel
Y' est O, S, ou NR', et
R' est un phényle, ou un biphénylyle.

11. Dispositif électroluminescent organique selon la revendication 1, dans lequel
Y' est CR'R", et
R' et R" sont un méthyle, ou R' et R" ensemble forment un cycle fluorène.

12. Dispositif électroluminescent organique selon la revendication 1, dans lequel
L' est une liaison simple, ou un phénylène.

13. Dispositif électroluminescent organique selon la revendication 1, dans lequel
le composé représenté par la Formule chimique 2 est un quelconque sélectionné à partir du groupe consistant en les suivants :
